# EUROPEAN PATENT APPLICATION

(11) **EP 4 606 370 A1**
(43) Date of publication of application: **27.08.2025**
(21) Application number: 23879889.6
(22) Date of filing: 20.10.2023
(51) Int. Cl.: A61K 9/14, A61K 9/127, A61K 9/51, A61K 45/00, A61K 45/06, A61K 47/26, A61K 47/44, A61K 47/46, A61P 35/00

(54) **METHOD FOR FUSING LIPID NANOPARTICLES**

(30) Priority: 21.10.2022 JP 2022168904
(71) Applicant: Craif Inc., Tokyo 113-0034 (JP)
(72) Inventor: YASUI, Takao, Tokyo 152-8550 (JP); BABA, Yoshinobu, Nagoya-shi, Aichi 464-8601 (JP); KAWAGUCHI, Shota, Kiyosu-shi, Aichi 452-0961 (JP)
(74) Representative: Michalski Hüttermann & Partner Patentanwälte mbB
(86) International application number: PCT/JP2023/038017
(87) International publication number: WO 2024/085247

(57) **Abstract**

According to one embodiment of the present disclosure, a method for fusing lipid nanoparticles is provided. In some embodiments, the method is a method including: causing a fibrous structure to capture a first lipid nanoparticle; causing the fibrous structure to capture a second lipid nanoparticle; and causing the fibrous structure to contract.

## Description

### TECHNICAL FIELD

The present disclosure relates to a method for fusing lipid nanoparticles.

### BACKGROUND ART

Technologies for artificially fusing lipid nanoparticles have been developed. In particular, the fusion of lipid nanoparticles has been developed for many years as an effective drug delivery system (DDS) technology for pharmaceuticals.

For example, DDSs using liposomes or micelles have been developed. Liposomes themselves, however, are easily removed in tissues such as the liver and spleen; they are deficient in long-term stability in the blood. This can reportedly be overcome to some extent by modifying the surface of the liposomes with polyethylene glycol (PEGylation), but the problems stated below also exist. In addition, manufacturing liposome preparations requires special machinery.

Micellar nanoparticles generally have a size of 30 to 100 nm and reportedly improve the efficacy and safety of pharmaceuticals because they easily concentrate in diseased sites. Micellar nanoparticles, however, easily accumulate in specific organs, such as the liver and lungs; they are unlikely to have specificity for specific organs.

For such DDSs, too, there are expectations for improved efficiency in drug delivery. For example, the method of fusing the lipid bilayers of biological exosomes and liposomes, which are attracting attention as DDSs, by the freeze-thaw method has been reported (Reference Publication 3). Through the fusion, reportedly, the surface characteristics of the exosomes are modified. This leads to reduced immunogenicity and improved colloidal stability, resulting in an extended half-life of the exosomes in the blood. Fused exosomes, furthermore, exhibit improved efficiency in cellular uptake, suggesting their potential application to DDSs (NPL 1).

To accelerate the membrane fusion, calcium ions, for example, may be added in some cases. The surface of the lipid bilayers is covered with water molecules, and they cause a repulsive force to arise between the bilayers. Calcium ions are believed to replace the water molecules on the surface of the lipid bilayers, further destabilizing the lipid bilayers and thereby enabling contact between the lipid bilayers. The addition of calcium ions, however, also causes problems. For example, calcium ions contribute to other biological phenomena and, furthermore, can induce aggregation.

Moreover, polyethylene glycol (PEG), for example, may be added in some cases. PEG contributes to membrane fusion without causing problems such as influences on biochemical phenomena and aggregation. According to one reported hypothesis, PEG is capable of binding water molecules together, and liposomes modified with PEG help improve liposome retention in the blood and also help remove water molecules from the lipid bilayers. Utilizing the effect in which molecules leak out of vascular endothelial cells with increased permeability within tumor tissue and accumulate within the containing tissue (the enhanced permeability and retention (EPR) effect), the development of PEG-modified liposomes as a promising drug delivery system (DDS), for example for nucleic acid therapeutics, is ongoing. Modification with a large amount of PEG, however, is considered disadvantageous in that it limits contact with target cancer cells and thus reduces cellular uptake. To address this, various development attempts have been made. Some other methods are also known, but they require special or expensive equipment.

Lipid nanoparticles with the same sign of charge are unlikely to be fused because electrical repulsion acts between them.

### CITATION LIST

### NON PATENT LITERATURE

NPL 1: Y.T. Sato, K. Umezaki, S. Sawada, S. Mukai, Y. Sasaki, N. Harada, H. Shiku and K. Akiyoshi, Engineering hybrid exosomes by membrane fusion with liposomes. Scientific Reports. 6, 21933; doi: 10.1038/srep21933 (2016).

### SUMMARY OF INVENTION

According to one embodiment of the present disclosure, a method for fusing lipid nanoparticles is provided. This method includes causing a fibrous structure to capture a first lipid nanoparticle; causing the fibrous structure to capture a second lipid nanoparticle; and causing the fibrous structure to contract. The first lipid nanoparticle and the second lipid nanoparticle may be fused through the contraction of the fibrous structure. A fused lipid nanoparticle may be formed through it.

This enables the fusion of lipid nanoparticles with a high degree of flexibility, for example without being influenced by ions present around the lipid bilayers (e.g., calcium ions) or being restricted by conditions such as the need for modification, for example with PEG, or process temperature (the need for low-temperature treatment).

According to one embodiment of the present disclosure, in the method for fusing lipid nanoparticles;
the first lipid nanoparticle encapsulates a first substance;
the second lipid nanoparticle encapsulates a second substance; and
the fusing the first lipid nanoparticle and the second lipid nanoparticle (and thereby forming a fused lipid nanoparticle) by causing the fibrous structure to contract includes mixing the first substance and the second substance and/or allowing them to react.

This enables, for example, more efficient manufacturing of the desired DDS drug with a high degree of flexibility. In general, artificial lipid nanoparticles (LNPs) easily accumulate in the liver and are readily eliminated through immune responses. Fused lipid nanoparticles, by contrast, have lipid bilayers derived from the subject and are unlikely to induce immune responses. Fused lipid nanoparticles, therefore, are easily absorbed by tissues in the body and are well-suited for DDSs.

Further aspects and advantages of the present disclosure will become readily apparent to those skilled in the art from the following detailed description, in which only exemplary embodiments of the present disclosure are presented and described. As will be understood, other different embodiments of the present disclosure are possible, and some details thereof can be modified in various obvious respects without departing from the present disclosure. Accordingly, the drawings and description are to be considered illustrations in nature and not to be considered limitations.

### BRIEF DESCRIPTION OF DRAWINGS

[Fig. 1] Fig. 1 illustrates a flowchart of a method according to an embodiment for fusing lipid nanoparticles using a fibrous structure.
[Fig. 2] Fig. 2 includes schematic views for describing steps according to an embodiment for fusing lipid nanoparticles using a fibrous structure.
[Fig. 3] Fig. 3 presents the size distribution of LNPs in different states as measured by nano-tracking analysis in Example 1.
[Fig. 4] Fig. 4 presents side scatter versus fluorescence intensity histograms of LNPs in different states as measured by flow cytometry in Example 1.
[Fig. 5] Fig. 5 presents the fluorescence intensity of LNPs in different states as measured using a plate reader in Example 1.
[Fig. 6] Fig. 6 presents the surface potential of LNPs in different states in Example 1.
[Fig. 7] Fig. 7 presents the size distribution of LNPs in different states as measured by nano-tracking analysis in Example 2.
[Fig. 8] Fig. 8 presents a side scatter versus fluorescence intensity histogram of fused LNPs as measured by flow cytometry in Example 2.
[Fig. 9] Fig. 9 presents the fluorescence intensity of LNPs in different states as measured using a plate reader in Example 2.
[Fig. 10] Fig. 10 presents the surface potential of LNPs in different states in Example 2.
[Fig. 11] Fig. 11 presents the size distribution of LNPs in different states as measured by nano-tracking analysis in Example 3.
[Fig. 12] Fig. 12 presents a side scatter versus fluorescence intensity histogram of fused LNPs as measured by flow cytometry in Example 3.
[Fig. 13] Fig. 13 presents the fluorescence intensity of LNPs in different states as measured using a plate reader in Example 3.
[Fig. 14] Fig. 14 presents the surface potential of LNPs in different states in Example 3.
[Fig. 15] Fig. 15 presents the size distribution of EVs before fusion as measured by nano-tracking analysis in Example 4.
[Fig. 16] Fig. 16 presents the size distribution of fused LNPs as measured by nano-tracking analysis in Example 4.
[Fig. 17] Fig. 17 presents side scatter versus fluorescence intensity histograms of fused LNPs as measured by flow cytometry in Example 4.
[Fig. 18] Fig. 18 presents the surface potential of LNPs in different states in Example 4.
[Fig. 19] Fig. 19 includes schematic views for describing steps according to an embodiment for fusing lipid nanoparticles using a fibrous structure.
[Fig. 20] Fig. 20 presents electron micrographs of different types of LNPs in Example 5.
[Fig. 21] Fig. 21 presents the size distribution of different types of LNPs as measured by nano-tracking analysis in Example 5.
[Fig. 22] Fig. 22 presents side scatter versus fluorescence intensity histograms of different types of LNPs as measured by flow cytometry in Example 5.
[Fig. 23] Fig. 23 presents the fluorescence intensity of different types of LNPs as measured using a plate reader in Example 5.
[Fig. 24] Fig. 24 presents the surface potential of different types of LNPs in Example 5.
[Fig. 25] Fig. 25 is a graph illustrating, as an example, the effects of the addition of miR1246 and an IL mixture on IL6 expression.
[Fig. 26] Fig. 26 presents side scatter versus fluorescence intensity histograms of different types of LNPs as measured by flow cytometry in Example 6.
[Fig. 27] Fig. 27 includes schematic views for describing steps according to an embodiment for fusing lipid nanoparticles using a fibrous structure.
[Fig. 28] Fig. 28 presents histograms of fluorescence intensity from membrane staining of fused LNPs compared with the fluorescence intensity of a fluorophore in a molecular beacon as measured by flow cytometry in Example 7.
[Fig. 29] Fig. 29 presents histograms of fluorescence intensity from membrane staining of fused LNPs compared with the fluorescence intensity of a fluorophore in a molecular beacon as measured by flow cytometry in Example 8.

### DESCRIPTION OF EMBODIMENTS

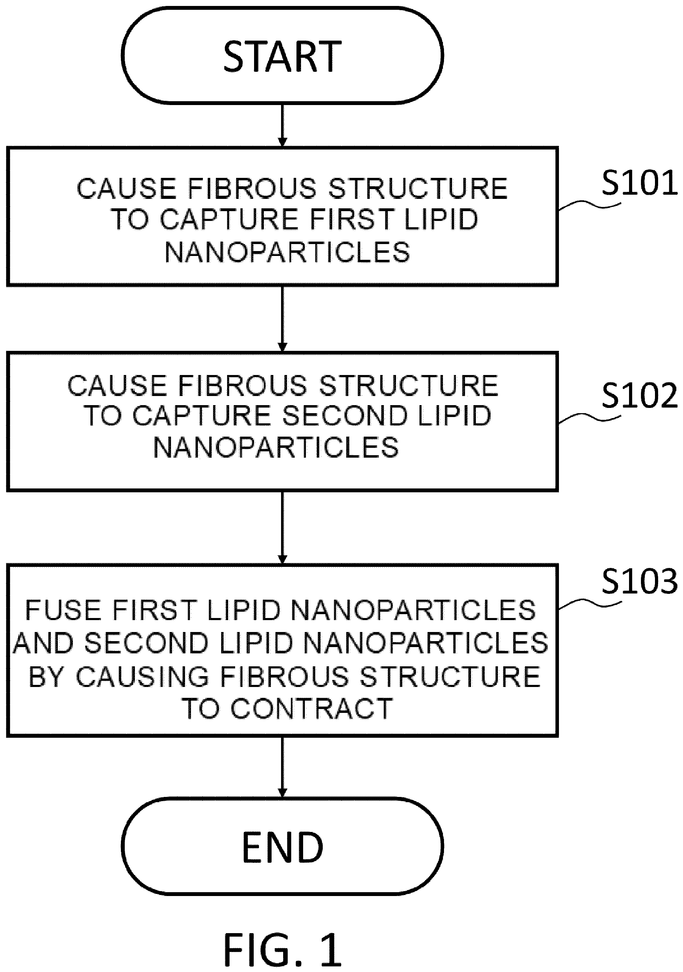

In Fig. 1, the process flow of a method according to an embodiment of the present disclosure for fusing lipid nanoparticles is illustrated. A fibrous structure is caused to capture first lipid nanoparticles (S101). The fibrous structure is caused to capture second lipid nanoparticles (S102). With the fibrous structure capturing the first lipid nanoparticles and the second lipid nanoparticles, the first lipid nanoparticles and the second lipid nanoparticles are fused by causing the fibrous structure to contract (S103).

Causing the fibrous structure to capture the first lipid nanoparticles (S101) and causing the fibrous structure to capture the second lipid nanoparticles (S102) may be in the reverse order of that illustrated in Fig. 1 or may be simultaneous. That is, in some embodiments, a fibrous structure may be caused to capture first lipid nanoparticles (S101), and then the fibrous structure may be caused to capture second lipid nanoparticles (S102). In some embodiments, a fibrous structure may be caused to capture second lipid nanoparticles (S102), and then the fibrous structure may be caused to capture first lipid nanoparticles (S101). In some embodiments, a fibrous structure may be caused to capture first lipid nanoparticles and second lipid nanoparticles substantially simultaneously.

Causing the fibrous structure to capture lipid nanoparticles may be, or may include, allowing a solution containing the lipid nanoparticles to be absorbed or infiltrate into the fibrous structure.

### <Lipid Nanoparticles>

"Lipid nanoparticles" (LNPs) used herein is used interchangeably with solid lipid nanoparticles (SLNPs/SLNs). In general, it refers to nanometer-sized particles composed of lipids.

Lipid nanoparticles may be biomolecules of natural origin or biomolecules collected from an animal. Lipid nanoparticles may be cell organelles or may be vesicles. Vesicles may non-limitingly be, for example, vacuoles, lysosomes, transport vesicles, secretory, gas vesicles, extracellular matrix vesicles, or extracellular vesicles or may include multiple types of them. Extracellular vesicles (EVs) may non-limitingly be, for example, exosomes, exosome complexes, ectosomes, shedding microvesicles, microvesicles, membrane particles, plasma membranes, or apoptotic blebs.

Lipid nanoparticles may non-limitingly be cells or may include cells. The cells may be, for example, erythrocytes, leukocytes, or immunocytes. Lipid nanoparticles may be, for example, viruses or bacteria.

Lipid nanoparticles may be liposomes of natural origin or artificially manufactured liposomes.

Examples of combinations of lipid nanoparticles to be fused non-limitingly include the following: liposomes and liposomes; liposomes and extracellular vesicles; liposomes and cells; liposomes and viruses; extracellular vesicles and extracellular vesicles; extracellular vesicles and cells; extracellular vesicles and viruses; cells and cells; cells and viruses; and viruses and viruses. The combinations of liposomes and liposomes; extracellular vesicles and extracellular vesicles; cells and cells; and viruses and viruses may consist of two groups of the same type or may consist of two different types.

In some embodiments, one group (the first or second) of lipid nanoparticles may be derived from a subject or patient (autologous lipid nanoparticles), and the other group (the second or first) of lipid nanoparticles may be non-autologous (allogenic or artificial) lipid nanoparticles. As a nonlimiting example, the first lipid nanoparticles may be exosomes derived from a subject or patient, and the second lipid nanoparticles may be artificial liposomes.

### <Substances Encapsulated in Lipid Nanoparticles and Substances That Lipid Nanoparticles Have in Their Membrane>

Lipid nanoparticles may contain a target substance or other substances. For example, extracellular vesicles, such as exosomes, contain lipids, proteins, and other substances derived from cell membranes in their surface and contain substances such as nucleic acids (e.g., microRNA, messenger RNA, and DNA), proteins, and body fluids inside.

The nucleic acids may be ribonucleic acids (RNAs) or may include ribonucleic acids (RNAs). The RNAs may non-limitingly be, for example, messenger RNAs (mRNAs), transfer RNAs (tRNAs), ribosomal RNAs (rRNAs), non-coding RNAs (ncRNAs), microRNAs (miRNAs), ribozymes, double-stranded RNAs (dsRNAs), or siRNAs (small interfering RNAs) or may include multiple types of them. The RNAs may be modified. The RNAs or miRNAs may be involved in the development and progress of, for example, cancers, cardiovascular diseases, neurodegenerative diseases, psychiatric diseases, or chronic inflammatory diseases. The miRNAs may be RNAs of the type that promotes, or positively regulates, carcinogenesis (onco miRNAs (oncogenic miRNAs)) or may be RNAs of the type that suppresses, or negatively regulates, carcinogenesis (Tumor Suppressor miRNAs).

The nucleic acids may be deoxyribonucleic acids (DNAs) or may include DNAs.

In some embodiments, lipid nanoparticles to be fused may contain a drug (e.g., an anticancer agent, a nucleic acid therapeutic, an anti-aging substance, or an anesthetic) inside, and the lipid nanoparticles formed through fusion (fused lipid nanoparticles) may contain this drug inside. In some embodiments, such fused lipid nanoparticles can be used as a DDS drug. In some embodiments, the fused lipid nanoparticles can be used as an anticancer agent.

In some embodiments, lipid nanoparticles may encapsulate a substance that recognizes a substance encapsulated in the counterpart lipid nanoparticles to be fused (target lipid nanoparticles) and binds to or reacts with it. For example, lipid nanoparticles may contain a nucleic acid or a molecular beacon having its nucleic acid sequence that hybridizes with a nucleic acid encapsulated in exosomes or other extracellular vesicles as the counterpart lipid nanoparticles (target lipid nanoparticles). This enables, for example, the detection or analysis of a nucleic acid present within a subject's lipid nanoparticles. In addition, this enables the estimation or acquisition of biological information of the subject (e.g., the type, presence or absence, status, future risks, and site of a disease).

**In** some embodiments, one group of lipid nanoparticles to be fused (e.g., the second lipid nanoparticles) has a protein on its membrane and undergoes fusion with the other group of lipid nanoparticles (e.g., the first lipid nanoparticles). The fused lipid nanoparticles have, on their membrane, the protein originally present on the first lipid nanoparticles and the protein originally present on the second lipid nanoparticles. For example, the protein on the membrane of the second lipid nanoparticles may be PEG. **In** some embodiments, one group of lipid nanoparticles to be fused may have an inorganic substance. The inorganic substance may be a substance, such as nanodiamonds, used in quantum sensors and/or quantum dots.

In some embodiments, one group of lipid nanoparticles to be fused may have a cancer-targeted ligand on its membrane. Fused lipid nanoparticles made using it may non-limitingly be used as, for example, active-targeting lipid nanoparticles.

### <Lipid Nanoparticle Solution>

Lipid nanoparticles may be provided as contained in a solution or liquid. A solution containing lipid nanoparticles may be introduced, absorbed, and allowed to infiltrate into the fibrous structure. "Solution" used herein includes a liquid containing a target substance, a liquid considered to contain a target substance, or a liquid that is used for the purpose of causing a target substance to be captured in the fibrous structure or for related purposes, even if it contains no target substance (Hereinafter, such a solution may also be referred to as "a solution containing lipid nanoparticles. ").

In some embodiments, a first solution containing the first lipid nanoparticles and a second solution containing the second lipid nanoparticles may be separately provided or introduced into the fibrous structure. In some embodiments, the first solution and the second solution may be introduced into the fibrous structure simultaneously or at different times. The first solution and the second solution may be provided as the same solution. That is, a certain solution may contain the first lipid nanoparticles and the second lipid nanoparticles. In some embodiments, a solution containing both the first lipid nanoparticles and the second lipid nanoparticles may be provided.

A solution containing lipid nanoparticles may be a body fluid or a liquid (e.g., a diluted liquid or processed liquid) derived from a body fluid. The solution may be a solution that is not a body fluid (not derived from a body fluid), may be a liquid that has been artificially prepared, or may be a mixture of a body fluid or a solution derived from a body fluid and a solution not derived from a body fluid. The solution may be a solution for use in sample measurement or may be a solution for use in calibration measurement. The solution may be used as a stock solution or may be a liquid that has been diluted or concentrated from a stock solution. The solution may be a standard solution or a calibration solution. The sample for measurement may be a biospecimen. The solution may contain a physiological buffer, such as phosphate buffered saline (PBS) or N-tris(hydroxymethyl)methyl-2-aminoethanesulfonic acid buffer (TES), containing a substance to be collected. The body fluid may contain additives. To the additives, a stabilizer and a pH-adjusting agent, for example, may have been added.

The body fluid may non-limitingly be blood, serum, plasma, or lymph, may be tissue fluid, such as interstitial tissue fluid, intercellular fluid, or interstitial fluid, or may be coelomic fluid, chorionic cavity fluid, pleural fluid, abdominal fluid, pericardial fluid, cerebrospinal fluid (spinal fluid), joint fluid (synovial fluid), or aqueous humor (humor aquosus). The body fluid may be a digestive juice, such as saliva, gastric juice, bile, pancreatic juice, or intestinal juice, or may be sweat, tears, nasal mucus, urine, seminal fluid, vaginal fluid, amniotic fluid, or milk.

The body fluid may be a human body fluid. The body fluid may be an animal body fluid. The animal may be a reptile, a mammal, or an amphibian. The mammal may be a canine, a feline, a bovine, an equine, an ovine, a porcine, a hamster, a mouse, or a squirrel or a primate, such as a monkey, a gorilla, a chimpanzee, a bonobo, or a human.

In some embodiments, a solution of lipid nanoparticles may be an aqueous solution. In some embodiments, a solution of lipid nanoparticles may be a nonaqueous solution.

<Fibrous Structure>

The term "fibrous structure" used herein refers generally to a component having pores and formed by mechanically and/or chemically processing fiber. Fibrous structures include, for example, a textile, a nonwoven fabric, and paper. The fibrous structure in the present disclosure preferably has hydrophilicity sufficient for an aqueous solution to penetrate it via capillary action or a higher degree of hydrophilicity. The fibrous structure may be formed in the shape of a sheet, or a flat shape (also referred to as a fiber sheet). Herein, a significant part of the description of the fibrous structure uses the example of a fiber sheet. The present disclosure, however, is not limited to sheet-shaped structures; fibrous structures substantially in a three-dimensional shape are also included.

In some embodiments, the fiber may be a chemical fiber, a natural fiber, or a fiber obtained by blending multiple types of fibers.

In some embodiments, the natural fiber may be a plant fiber, such as cellulose, cotton, hemp, or linen. In some embodiments, it may be an animal fiber, such as wool, silk, or cashmere.

The term "cellulose fiber structure" used herein refers generally to a fibrous structure primarily composed of cellulose fibers or containing cellulose fibers. The fibrous structure cellulose fiber structure may be a cellulose fiber sheet. "Cellulose fiber" used herein refers to a fiber primarily composed of cellulose. The cellulose fibers may be cellulose nanofibers (CNFs). In general, cellulose nanofibers have a diameter of nanometers (nm) to tens of nm. In many cases, as a nonlimiting example, the width of cellulose nanofibers is from approximately 3 nm to 100 nm.

Cellulose fibers can be chemically and/or mechanically acquired from, for example, wood pulp or other source materials. A typical method for manufacturing cellulose nanofibers is as follows. First, wood fibers (cellulose fibers) are extracted from wood chips and processed into pulp. These cellulose fibers are composed of bundles of numerous cellulose nanofibers. Then these cellulose fibers are subjected to collisions between fibers under high pressure in a solvent in the presence of a TEMPO catalyst to unravel the bundled cellulose fibers. Through this, cellulose nanofibers can be obtained. This method for manufacturing cellulose fibers and cellulose nanofibers is a nonrestrictive example; another manufacturing method may be employed.

Suction-filtering the resulting solvent containing cellulose nanofibers causes the cellulose nanofibers to aggregate or form a film due to surface tension. The cellulose nanofiber solvent may be, for example, water. In a certain configuration, the produced film can be a nonwoven fabric.

For nanopores, a liquid with low surface tension (Hereinafter, this liquid may be written as "the low-surface-tension solvent.") is added to the cellulose nanofibers in the wet state that have aggregated through suction filtration. The suction of the nanofibers is continued, thereby allowing the solvent contained in the aggregated mass of cellulose nanofibers to be replaced with the low-surface-tension solvent or dried. Through this, nanopores are created inside the assembly of cellulose nanofibers.

The size of the nanopores can be adjusted by, for example, the low-surface-tension solvent added. The surface tension of the low-surface-tension solvent may be lower than the surface tension of water (20°C, 72.75 mN/m) and fall within a range that allows for nanopore production. For example, the surface tension of the solvent at 20°C may be equal to or lower than values such as 35 mN/m, 30 mN/m, 25 mN/m, and 20 mN/m. Examples of low-surface-tension solvents non-limitingly include tertiary butyl alcohol (20.7 mN/m), ethanol (22.55 mN/m), and isopropanol (20.8 mN/m).

These methods for the formation of nanopores and the adjustment of their size are examples; methods other than these may be used. For example, the high-pressure treatment conditions under which the cellulose fibers are unraveled may be employed. For example, the type of pulp may be changed. For example, cellulose derived from a different source (examples include acetic acid bacteria (e.g., *Acetobacter*) and other microorganisms; and animals, such as sea squirts) may be used. The size of the nanopores may be adjusted by changing the width of the cellulose nanofibers by these approaches.

In some embodiments, the fibrous structure may be formed of cellulose fibers (pulp). By dispersing cellulose fibers (pulp) in a solvent, a fibrous structure can be manufactured substantially in the same manner as in the case of cellulose nanofibers.

The gaps between cellulose fibers (pore size) can be adjusted substantially in the same manner as in the case of cellulose nanofibers. The width (diameter) of cellulose fibers is from approximately 20 µm to 40 µm in many cases. The size of the gaps, therefore, is from several nm to several µm and may be from approximately 10 nm to approximately 1000 nm or from approximately 1 µm to 100 µm.

In some embodiments, the fiber may be a chemical fiber. In some embodiments, the chemical fiber may be a polymeric fiber or may be a synthetic fiber of polyvinyl alcohol (such as PVA). The chemical fiber may be, for example, a polyester fiber, such as polyethylene terephthalate, polynaphthalene terephthalate, polyethylene naphthalate, or polytrimethylene terephthalate; a polyamide fiber, such as nylon; an acrylic fiber, such as acrylonitrile; a polyolefin fiber, such as polyethylene or polypropylene; a recycled cellulose fiber, such as rayon, cuprammonium rayon, or polynosic; a cellulose-based semisynthetic fiber, such as cellulose acetate; a protein-based semisynthetic fiber, such as promix; a polyurethane fiber; a vinylon fiber; glass fiber; or carbon fiber (Carbon nanotubes are also included.). In some embodiments, the fiber may be a hydrolyzable polymer, such as polydioxanone (PDO or PDS), a biodegradable polymer, an absorbable polymer, or an absorbable suture.

"Pore size" or "feature size" used herein refers to the size of pores in a fibrous structure. The pore size may be a value such as 2 nm, 3 nm, 4 nm, 5 nm, 6 nm, 7 nm, 8 nm, 9 nm, 10 nm, 15 nm, 20 nm, or 30 nm or greater than it. The pore size may be a value such as 20 nm, 30 nm, 40 nm, 50 nm, 60 nm, 70 nm, 80 nm, 90 nm, 100 nm, 150 nm, 200 nm, 300 nm, 400 nm, or 500 nm or smaller than it. The pore size may fall within a range such as from approximately 4 nm to approximately 200 nm, from approximately 40 nm to approximately 200 nm, from approximately 200 nm to approximately 500 nm, from approximately 4 nm to approximately 100 nm, from approximately 5 nm to approximately 90 nm, or from approximately 10 nm to approximately 80 nm.

For example, a fibrous structure having a pore size of approximately 40 nm to approximately 200 nm is suitable for capturing small EVs having a similar size. For example, a fibrous structure having a pore size of approximately 200 nm to approximately 500 nm is suitable for capturing large EVs having a similar size. These are examples; a fibrous structure having a different pore size may be used to capture EVs of such sizes.

### <Contraction of the Fibrous Structure>

In some embodiments, causing the fibrous structure to contract may include removing, from the fibrous structure, at least part of the water it retains. In some embodiments, causing the fibrous structure to contract may include removing solutions containing lipid nanoparticles. In some embodiments, causing the fibrous structure to contract may include drying. For example, the fibrous structure with solutions containing lipid nanoparticles absorbed in it may be dried and/or heated. Through this, the solutions or solvents can be evaporated.

In some embodiments, the fibrous structure may be configured such that during its manufacture or when absorbing a target solution, the target substance (lipid nanoparticles) can substantially enter or pass through gaps in the fibrous structure, and when the fibrous structure is washed (after drying or before washing), the target substance (lipid nanoparticles) is substantially retained by the structure and/or prevented from flowing out.

Causing the fibrous structure to contract causes the pore size of the fibrous structure to decrease. Preferably, the fibrous structure has been manufactured such that the pore size is comparable to or greater than the size of the target substance (lipid nanoparticles) to be captured in the wet state and is smaller than the size of the target substance after drying.

Multiple lipid nanoparticles originally captured in the same pore or closely spaced pores in the fibrous structure come into contact due to a decrease in pore size, or a decrease in the gap between fibers. The nanoparticles press one another, thereby fusing. Two lipid nanoparticles fuse, and the substances originally contained in their respective interior mix together.

### <Embodiment 1>

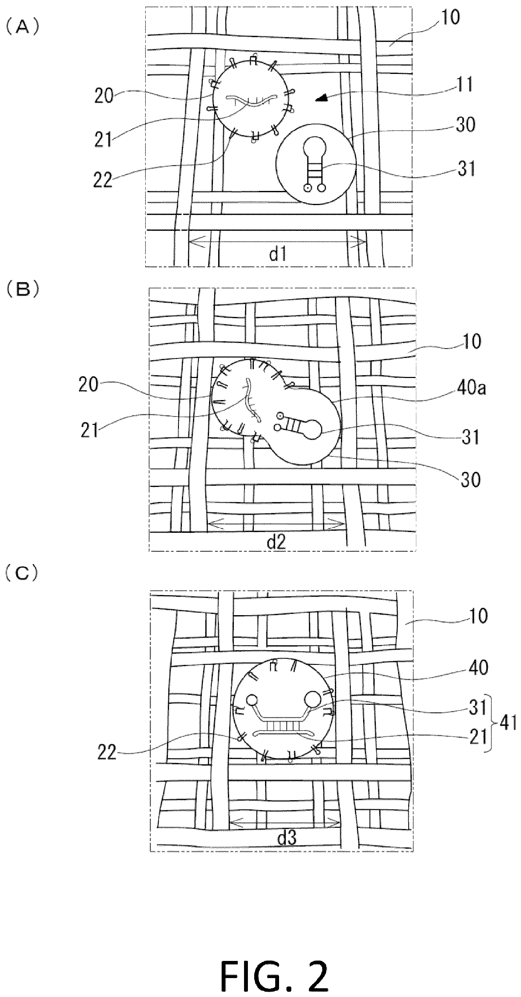

With reference to Fig. 2, a process according to an example of the fusion of lipid nanoparticles through the contraction of a fibrous structure and the mixing and reaction between substances therein is schematically described.

As illustrated in Fig. 2A, the fibrous structure 10 holds a first lipid nanoparticle 20 and a second lipid nanoparticle 30 captured in its pore 11. The size of the pore 11 is schematically indicated by d1 and is sufficiently large for the two lipid nanoparticles 20 and 30 to be embraced. The size of the pore 11 or the nature of the surface of the fibers in the fibrous structure 10, furthermore, allows the structure to continue holding the two lipid nanoparticles 20 and 30 without releasing them.

In the example in Fig. 2, the first lipid nanoparticle 20 is an exosome. Exosomes encapsulate various substances inside. In Fig. 2, one RNA 21 is illustratively presented. In Fig. 2, the exosome 20 illustratively holds expressed proteins 22 on its surface. The second lipid nanoparticle 30 is an artificially prepared liposome and contains a molecular beacon 31 inside that is designed to bind to the RNA 21 encapsulated in the exosome 10.

The d1 to d3 presented in Fig. 2 schematically indicate the distance between the two fibers that sandwich the captured lipid nanoparticle(s) and extend substantially in parallel. They are illustrative parameters presented for the description of changes in the size of the pore 11 in the schematic view of Fig. 2 and should not be construed as limiting. They may be other schematic parameters.

As illustrated in Fig. 2B, gradually drying the fibrous structure 10 causes the solvents to evaporate and the size of the pore 11 to decrease (d2 < d1). The two lipid nanoparticles, the exosome 20 and the liposome 30, are compressed from the outside by the fibers in the fibrous structure 10. The nanoparticles come into contact with each other, initiating fusion and starting to form a fused lipid nanoparticle 40a. Upon membrane fusion, the substances originally present inside the two lipid nanoparticles start mixing.

As illustrated in Fig. 2C, drying the fibrous structure 10 causes the size of the pore 11 to decrease (d3 < d2). Consequently, the exosome 20 and the liposome 30 completely fuse, forming a fused lipid nanoparticle 40.

Inside the fused lipid nanoparticle 40, the molecular beacon 31 originally encapsulated in the liposome 30 binds to the target RNA 21 originally encapsulated in the exosome 20. As a result, the fluorophore in the molecular beacon 31 leaves the quencher, emitting fluorescence (Fig. 2C).

The membrane of the fused lipid nanoparticle 40 holds proteins derived from the exosome 20 (Fig. 2C). The membrane of the fused lipid nanoparticle 40, furthermore, also holds proteins derived from the liposome 30 (not illustrated).

### <EXAMPLES>

Several examples will now be described. The procedure for the formation and fusion of LNPs was the same in all examples and was as follows.

### <LNP Formation>

The LNPs were produced using an iLiNP (invasive lipid nanoparticle production) device. The lipid solutions for anionic and cationic LNPs were 5 mM DSPC/DOPS/Chol (45/10/45 mol%) in EtOH:MeOH = 8:2 and 10 mM DOTAP/DSPC/Chol/PEG-amine (50/10/38.5/1.5 mol%) in EtOH:MeOH = 8:2, respectively. The aqueous solution contained a 13.4 mM CaCl₂/10 mM Tris-HCl (pH 8.0) buffer and 10 µM of molecules to be encapsulated. The lipid solution and the aqueous solution were simultaneously introduced into the device at a flow rate ratio of 2:1. The produced LNPs were subjected to dialysis at 4°C in PBS overnight to remove molecules present outside the LNP membrane.

To cellulose nanofibers (1 cm × 1 cm), 10 µL of a suspension of LNPs encapsulating a molecular beacon (labeled LNPs) was applied. The nanofibers were placed in a desiccator and dried for 3 days. Then 10 µL of a suspension of LNPs or EVs encapsulating an miRNA (target LNPs) was applied, and the nanofibers were again placed in a desiccator and dried for 3 days. Through this, fusion was performed. After that, the cellulose nanofibers were placed in a tube containing PBS and vigorously stirred to extract the fused LNPs.

### <EXAMPLE 1: Fusion between Positively Charged LNPs and Positively Charged LNPs>

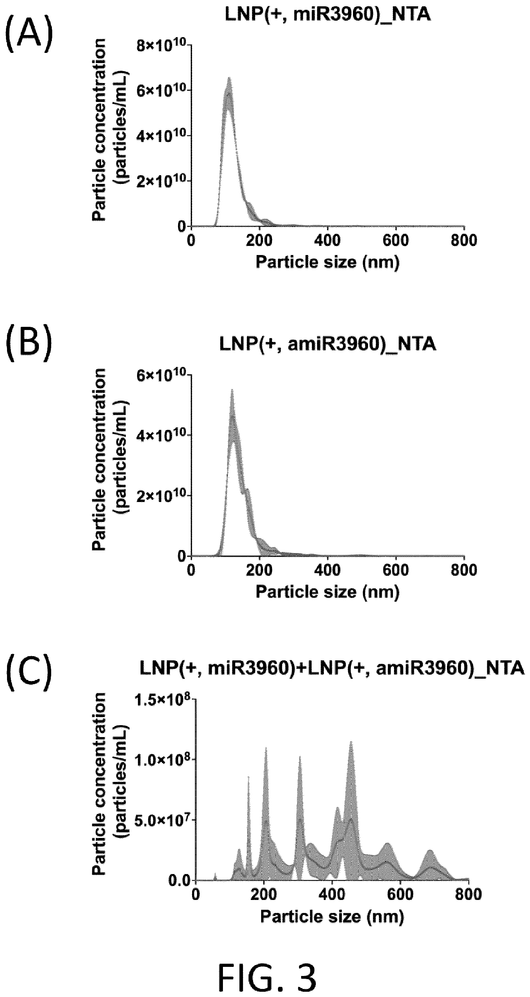

Two types of LNPs both having a positively charged surface (LNP(+)) were prepared. A detection miRNA (miR3960) was loaded into one type of LNP (target LNPs) (LNP(+, miR3960)). Using nano-tracking analysis (NTA, NanoSight, Malvern Instruments Ltd.), the size of the prepared LNPs was measured (Fig. 3A). It was confirmed that the particle size had a maximum at approximately 120 nm and ranged from 80 nm to 240 nm.

A molecular beacon designed to hybridize with the miR3960 for detection and having Cy5 as a fluorophore was loaded into the other type of LNP (labeled LNPs) (LNP(+, amiR3960)). The size of the prepared LNPs was measured in the same manner (Fig. 3B). It was confirmed that the particle size had a maximum at approximately 120 nm and ranged from 80 nm to 240 nm. Although some differences are present, the distribution presented in Fig. 3B can be considered substantially the same as that in Fig. 3A.

An aqueous solution containing both types of LNPs was absorbed into a cellulose fiber sheet, and the two types of LNPs were fused to form fused LNPs by a method according to the present disclosure. Then the fused LNPs were collected from the cellulose fibers. The size of the collected fused LNPs was measured (Fig. 3C). The size of the prepared LNPs was substantially undetectable. Instead, multiple peaks were observed over the range of 200 nm to 700 nm. This suggests that not only two, but a greater number of LNPs were also fused through the fusion process.

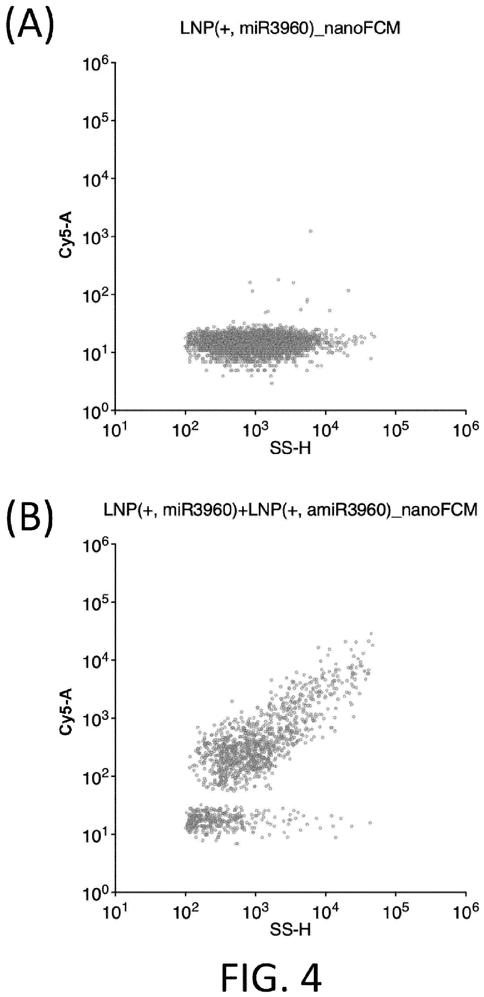

In Fig. 4, the results of flow cytometry for the target LNPs (Fig. 4A) and the fused LNPs (Fig. 4B) are presented. The flow cytometry was performed using NanoAnalyzer (nanoFCM). The horizontal axis (SS-H) represents side scatter intensity. It provides information about particle size. The vertical axis (Cy5-A) indicates fluorescence intensity.

In Fig. 4A, almost all measurement points were below at a fluorescence intensity of about 2 × 10¹. These points represent data that were counted as particles but not counted for the optical label. That is, they represent noise unrelated to the molecular beacon Cy5, which was the target for measurement. In Fig. 4B, by contrast, two distributions clearly separated at a fluorescence intensity of about 2 × 10¹ were present. The lower distribution represents noise corresponding to the distribution in Fig. 4A. The upper distribution is not noise; that is, it corresponds to the molecular beacon Cy5, which was the target for measurement.

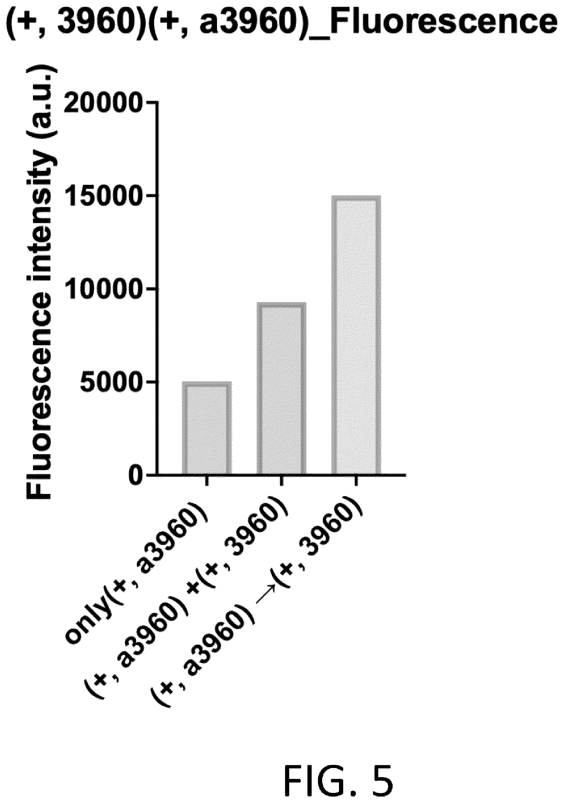

In Fig. 5, fluorescence intensity in each LNP state as measured using a plate reader is presented. A plate reader (Spark^{®} multimode microplate reader, TECAN) and plates (Thermo Scientific(TM) Black 96-Well Immuno Plates) were used. A 50-µL sample was dispensed dropwise per well, and fluorescence intensity was measured with Ex/Em = 630/675 (nm). The horizontal axis indicates each of the following cases: only the labeled LNPs that had yet to be fused were measured (only(+, a3960)); the labeled LNPs and the target LNPs were mixed first, then the mixture was introduced into cellulose nanofibers, and the fusion process was performed ((+, a3960)+(+, 3960)); and the labeled LNPs were introduced into cellulose nanofibers first, then the target LNPs were introduced into the cellulose nanofibers, and the fusion process was performed thereafter ((+, a3960)→(+, 3960)).

The fluorescence intensity of 5000 in the case of only the labeled LNPs that had yet to be fused (only(+, a3960)) can be considered noise or background.

The fluorescence intensity when the labeled LNPs and the target LNPs were mixed, the mixture was introduced into cellulose nanofibers, and the fusion process was performed ((+, a3960)+(+, 3960)) was 9000. This suggests that the labeled LNPs and the target LNPs fused through the fusion process.

The fluorescence intensity when the labeled LNPs were introduced into cellulose nanofibers first, then the target LNPs were introduced into the cellulose nanofibers, and the fusion process was performed thereafter ((+, a3960)→(+, 3960)) was 14000. This suggests that the labeled LNPs and the target LNPs fused. Fusion efficiency was high compared with that with introduction into cellulose fibers after mixing. The scientific reason for this is unclear at the time of the present application. While various mechanisms are possible, this trend should not be interpreted with limitation to a particular mechanism.

From these results, it is indicated that the molecular beacon originally present inside the labeled LNPs recognized the miR3960 originally present inside the target LNPs. That is, it was confirmed that the target LNPs and the labeled LNPs, which were both positively charged, were fused as a result of the fusion process.

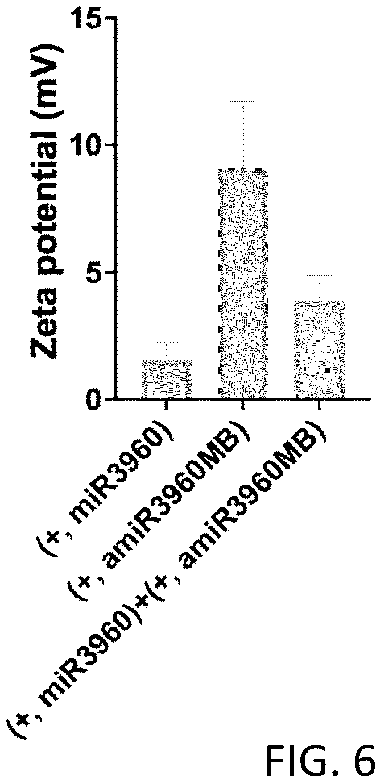

In Fig. 6, the results of surface potential (Zeta Potential (mV)) for each LNP state are presented. The surface potential was measured using a zeta potential analyzer (ELSZ-2000Z, Otsuka Electronics Co., Ltd.). The measurement was performed at room temperature using a standard cell. The horizontal axis indicates each of the following: the target LNPs that had yet to be fused ((+, miR3960)); the labeled LNPs that had yet to be fused ((+, amiR3960MB)); and the fused LNPs formed through mixing, subsequent introduction into cellulose fibers, and fusion ((+, miR3960)+(+, amiR3960MB)).

The surface potentials of the target LNPs before fusion ((+, miR3960)) and the labeled LNPs after fusion ((+, amiR3960MB)) were approximately 1 mV and approximately 9 mV, respectively. The surface potential of the fused LNPs after fusion ((+, miR3960)+(+, amiR3960MB)), on the other hand, was approximately 4 mV. That is, the charge on the fused LNPs has a value that is an average of the two types of LNPs before fusion. These results suggest that the target LNPs and the labeled LNPs, both having positive charge, underwent membrane fusion through the fusion process.

### <EXAMPLE 2: Fusion between Negatively Charged LNPs and Negatively Charged LNPs>

In this example, two types of LNPs both having a negatively charged surface (LNP(-)) were prepared, and these LNPs were fused. A detection miRNA (miR21) was loaded into one type of LNP (target LNPs) (LNP(-, miR21)). A molecular beacon designed to hybridize with the miR21 for detection and having Cy5 as a fluorophore was loaded into the other type of LNP (labeled LNPs) (LNP(-, amiR21)). The conditions for the fusion process, NTA measurement, flow cytometry measurement, and surface potential measurement were the same as in Example 1.

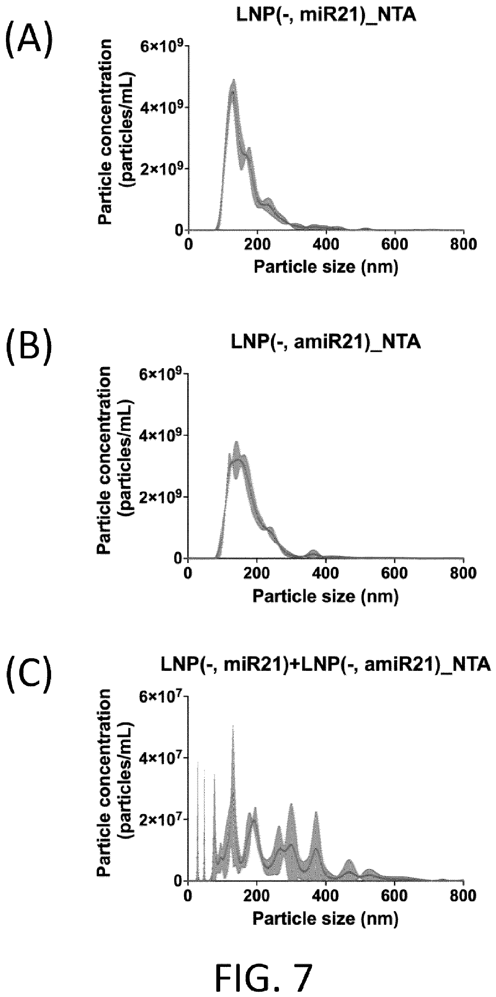

In Fig. 7, the size distribution of each type of LNP as measured using nano-tracking analysis is presented. It was confirmed that the size of the target LNPs and the labeled LNPs before fusion had a maximum at approximately 150 nm and ranged from 80 nm to 400 nm, although some differences were present (Figs. 7A and 7B).

As for the size of the fused LNPs, multiple new peaks were observed over the range of 150 nm to 600 nm, although a distribution was present at 150 nm.

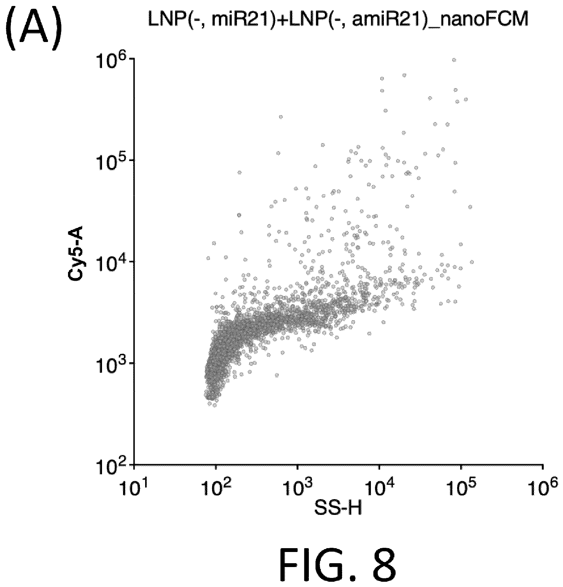

In Fig. 8, the results of flow cytometry for the fused LNPs are presented. A distribution was present at or above a Cy5-A of about 10⁴.

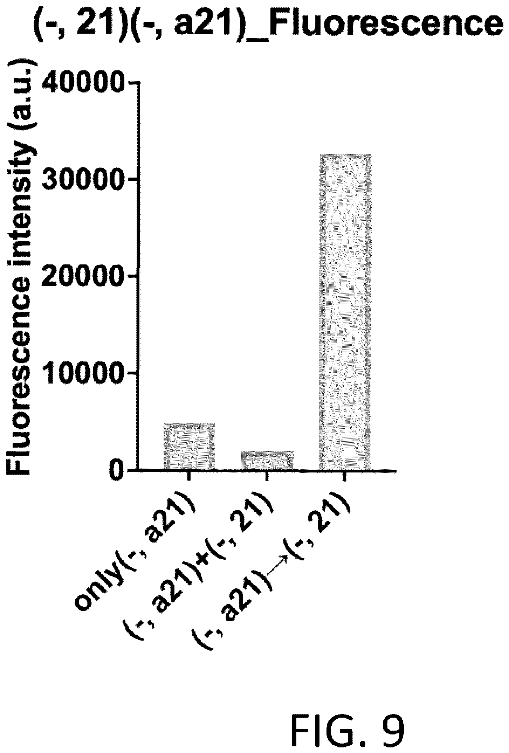

In Fig. 9, fluorescence intensity in each LNP state as measured using a plate reader is presented. The horizontal axis indicates each of the following cases: only the labeled LNPs that had yet to be fused were measured (only(-, a21)); the labeled LNPs and the target LNPs were mixed first, then the mixture was introduced into cellulose nanofibers, and the fusion process was performed ((-, a21)+(-, 21)); and the labeled LNPs were introduced into cellulose nanofibers first, then the target LNPs were introduced into the cellulose nanofibers, and the fusion process was performed thereafter ((-, a21)→(-, 21)).

The fluorescence intensity of 5000 for the labeled LNPs before fusion (only(-, a21)) can be considered noise or background. The fluorescence intensity after the mixing of the labeled LNPs and the target LNPs and the introduction of the mixture into cellulose nanofibers ((-, a21)+(-, 21)) was 2000. This suggests that the LNPs did not fuse. Unlike Example 1, in which both types of LNPs were negatively charged, no substantial fusion was observed. The fluorescence intensity after the fusion process ((-, a21)→(-, 21)) was 32000. This value is much higher than the other values, supporting that the molecular beacon bound to the target miRNA through the fusion process.

Between Example 1 and Example 2, the following difference was observed: two types of LNPs introduced into cellulose fibers after mixing fused when both were positively charged, but did not fuse when both were negatively charged. The scientific reason for this is unclear at the time of the present application. While various mechanisms are possible, this trend should not be interpreted with limitation to a particular mechanism.

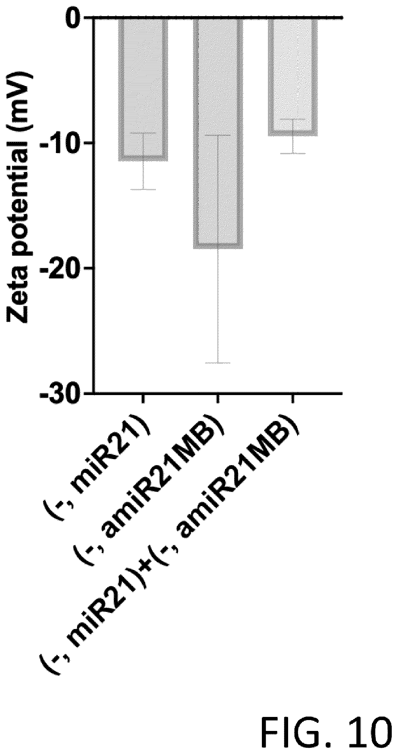

In Fig. 10, the results of surface potential (Zeta Potential (mV)) for each LNP state are presented. The horizontal axis indicates each of the following: the target LNPs that had yet to be fused ((-, miR21)); the labeled LNPs that had yet to be fused ((-, amiR21MB)); and the fused LNPs, which had been fused ((-, miR21)+(-, amiR21MB)).

The surface potentials of the target LNPs before fusion ((-, miR21)) and the labeled LNPs before fusion ((-, amiR21MB)) were approximately -10 mV and approximately -18 mV, respectively. The surface potential of the fused LNPs after fusion ((-, miR21)+(-, amiR21MB)), on the other hand, was approximately -9 m V. These results suggest that the target LNPs and the labeled LNPs, both having negative charge, underwent membrane fusion through the fusion process.

### <EXAMPLE 3: Fusion between Negatively Charged LNPs and Positively Charged LNPs>

In this example, LNPs having a negatively charged surface (LNP(-)) and LNPs having a positively charged surface (LNP(+)) were prepared, and these LNPs were fused.

A detection miRNA (miR3960) was loaded into one type of LNP, which was negatively charged LNPs (target LNPs) (LNP(-, miR3960)). A molecular beacon designed to hybridize with the miR3960 for detection and having Cy5 as a fluorophore was loaded into the other type of LNP (labeled LNPs) (LNP(+, amiR3960)). The conditions for the fusion process, NTA measurement, flow cytometry measurement, and surface potential measurement were the same as in Example 1.

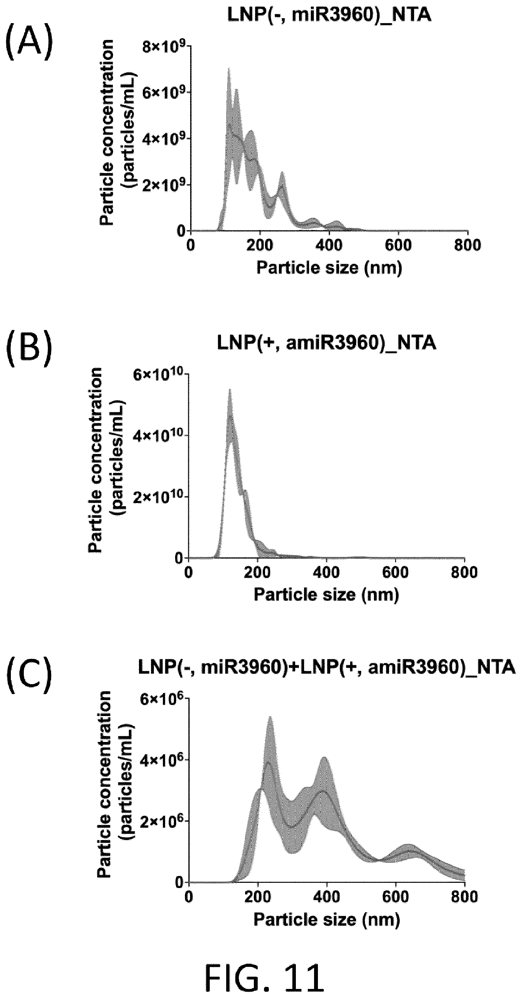

In Fig. 11, the size distribution of each type of LNP as measured using nano-tracking analysis is presented. It was confirmed that the size of the target LNPs and the labeled LNPs before fusion had a maximum at approximately 150 nm and ranged from 80 nm to 400 nm (Fig. 11A) and from 80 nm to 300 nm (Fig. 11B), although some differences were present.

As for the size of the fused LNPs, multiple new peaks were observed over the range of 150 nm to 800 nm (Fig. 1C).

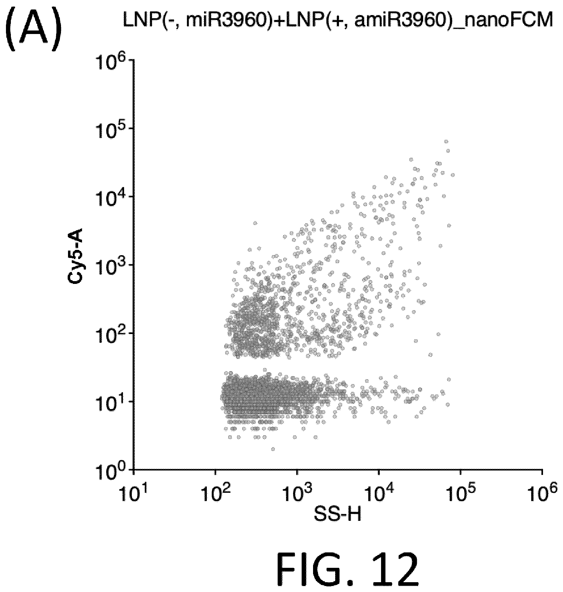

In Fig. 12, the results of flow cytometry for the fused LNPs are presented. The distribution above 2 x 10¹ is not noise but corresponds to the molecular beacon Cy5.

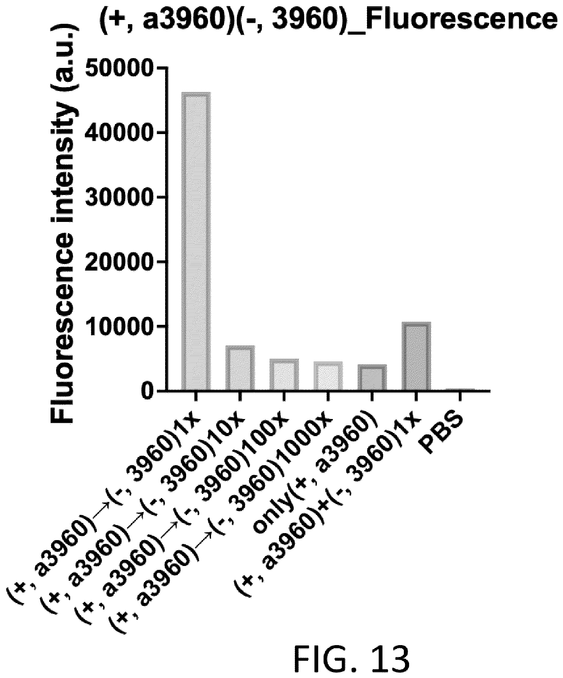

In Fig. 13, fluorescence intensity in each LNP state as measured using a plate reader is presented. The horizontal axis indicates each of the following cases: the target LNPs and the labeled LNPs were introduced in this order into cellulose nanofibers at a concentration ratio of 1:1, and the fusion process was performed ((+, a3960)→(-, 3960)1×); the concentration ratio was 10:1 ((+, a3960)→(-, 3960)10×); the concentration ratio was 100:1 ((+, a3960)→(-, 3960)100×); the same concentration ratio was 1000:1 ((+, a3960)→(-, 3960)1000×); only the labeled LNPs (only(+, a3960)); the target LNPs and the labeled LNPs were mixed at a concentration ratio of 1:1 first, then the resulting mixture was introduced into cellulose nanofibers, and the fusion process was performed ((+, a3960)+(-, 3960)1×); and only PBS was introduced into cellulose nanofibers (PBS).

In the case of dropwise addition in order ((+, a3960)→(-, 3960)1×), fusion efficiency was higher than when the LNPs were premixed (+, a3960)+(-, 3960)1×). In the group in which dropwise addition was performed in order, fluorescence intensity was the highest when the concentration of the labeled LNPs was 1:1, and fluorescence intensity decreased as the labeled LNPs were diluted. The inventors believe that this is because with dilution, the amount of the target miRNA (miR3960) decreased, and the molecular beacon encapsulated in the labeled LNPs was provided relatively excessively.

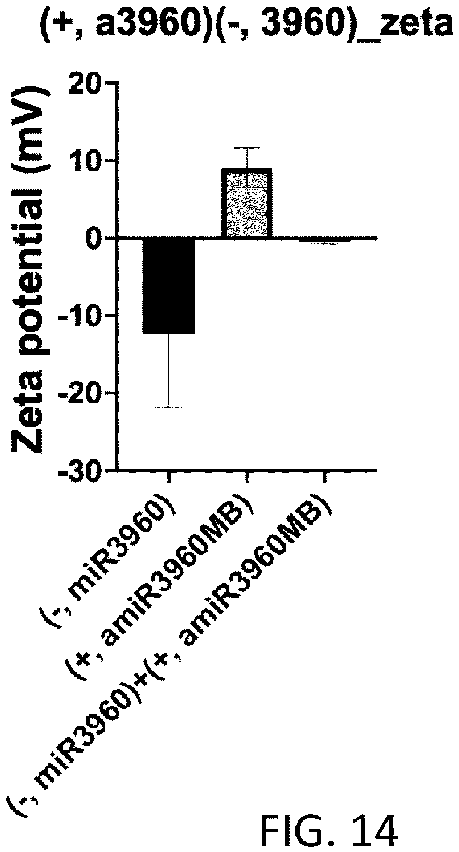

In Fig. 14, the results of surface potential (Zeta Potential (mV)) for each LNP state are presented. The horizontal axis indicates each of the following: the target LNPs, negatively charged, that had yet to be fused ((-, miR3960)), the labeled LNPs, positively charged, that had yet to be fused ((+, amiR3960MB)); and the fused LNPs formed through mixing, subsequent introduction into cellulose fibers, and fusion ((-, miR3960)+(+, amiR3960MB)).

The surface potentials of the target LNPs before fusion ((-, miR3960)) and the labeled LNPs after fusion ((+, amiR3960MB)) were approximately -12 mV and approximately +9 mV, respectively. The surface potential of the fused LNPs after fusion ((-, miR3960)+(+, amiR3960MB)), on the other hand, was approximately 0 mV. These results suggest that the target EVs, which had negative charge, and the labeled LNPs, which had positive charge, underwent membrane fusion through the fusion process.

### <EXAMPLE 4: Fusion Between EVs and LNPs>

In this example, EVs and LNPs having a positively charged surface (LNP(+)) were prepared, and these particles were fused.

Two types of EVs were prepared. One was extracellular vesicles derived from a normal cell strain without activation by an interleukin (EV(IL-)), and the other was extracellular vesicles derived from a normal cell strain activated by an interleukin (EV(IL+)).

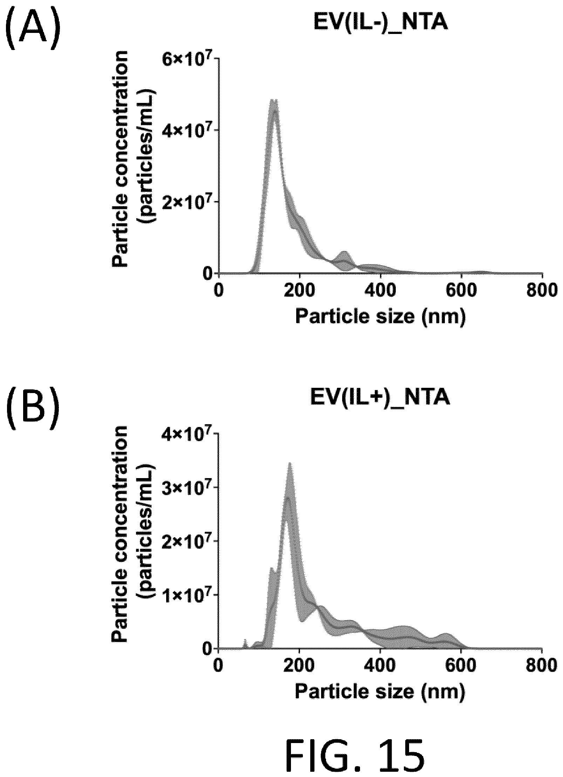

In Fig. 15, the size distribution of each type of EV as measured using nano-tracking analysis is presented. For the sizes of the target LNPs and the labeled LNPs before fusion, the following was confirmed. For EV(IL-), the distribution had a maximum at approximately 150 nm and ranged from 80 nm to 450 nm (Fig. 15A), and for EV(IL+), the distribution ranged from 80 nm to 600 nm (Fig. 15B).

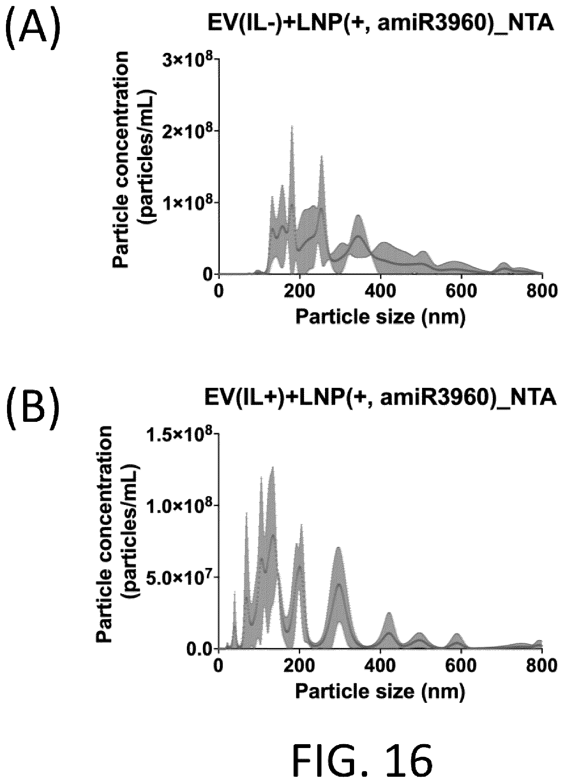

As for the size of the fused LNPs, multiple new peaks were observed over the range of 50 nm to 800 nm in both cases (Figs. 16A and 16B).

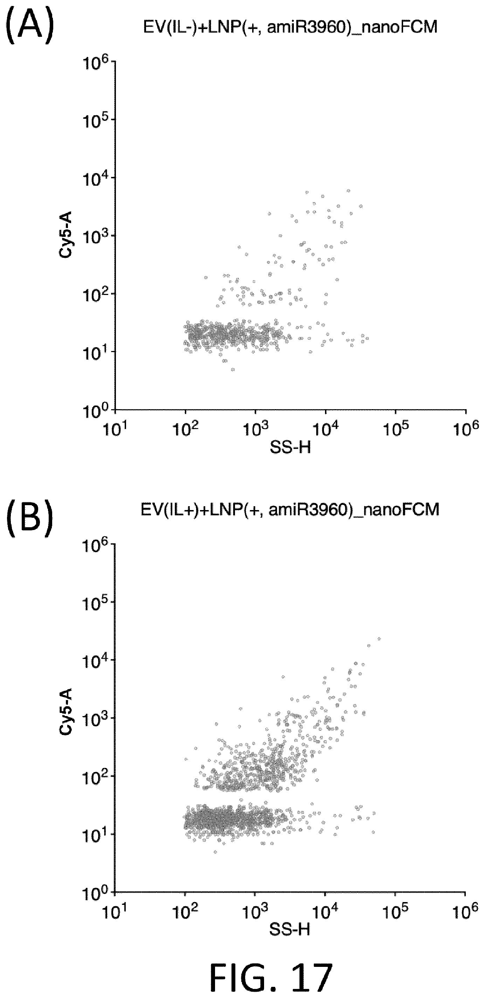

In Fig. 17, the results of flow cytometry for the fused LNPs are presented. The distributions above 2 x 10¹ are not noise but correspond to the molecular beacon Cy5.

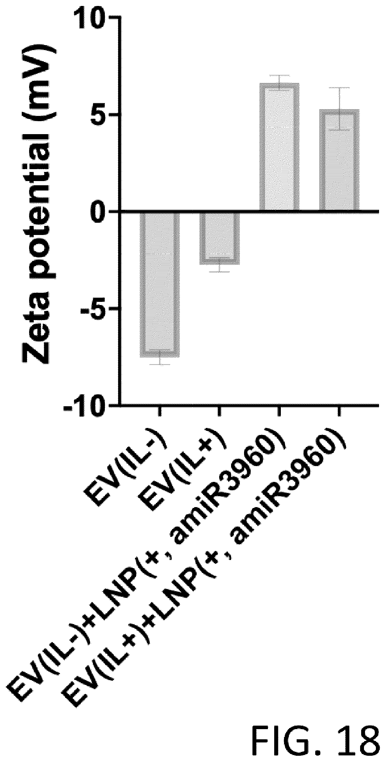

In Fig. 18, the results of surface potential (Zeta Potential (mV)) for each state of the EVs that had yet to be fused and the fused LNPs are presented. The horizontal axis indicates each of the following: target EVs (IL-) that had yet to be fused; target EVs (IL+) that had yet to be fused; fused LNPs after fusion with target EVs (IL-) (EV(IL-)+LNP(+, amiR3960)); and fused LNPs after fusion with target EVs (IL+) (EV(IL+)+LNP(+, amiR3960)).

The surface potentials of the target EVs (IL-) and the target EVs (IL-) before fusion were approximately -7.5 mV and approximately -2.5 mV, respectively. The surface potentials of the fused LNPs (EV(IL-)+LNP(+, amiR3960)) and the fused LNPs (EV(IL+)+LNP(+, amiR3960)) after fusion, on the other hand, were approximately +7 mV and approximately +5 mV, respectively. These results suggest that the target EVs, which had negative charge, and the labeled LNPs, which had positive charge, underwent membrane fusion through the fusion process.

Examples 1 to 4 demonstrate that target LNPs and labeled LNPs can be fused regardless of the sign of their charge, and that target LNPs and labeled LNPs having the same sign of charge, in particular, can be fused. These, furthermore, indicate that the contents of both can be mixed within the fused LNPs, and that the lipid membranes of both are obtained fused as the membrane of the fused LNPs.

### <Other Examples>

In some embodiments, the first LNPs may be derived from a subject, and the second LNPs may encapsulate a drug. The fusion process may be performed outside the body of the subject. The fused LNPs may be returned into the body of the subject. The subject may be a patient with cancer, and the drug may be an anticancer agent. In some embodiments, the first LNPs may be derived from a subject, and the second LNPs may encapsulate an anesthetic.

In some embodiments, the first LNPs (or the second LNPs) may be of biological origin, and the second LNPs (or the first LNPs) may be of biological origin or artificially synthesized ones and may be conditioned for the purpose of being fused with the first LNPs (or the second LNPs).

In some embodiments, first LNPs derived from a subject and the second LNPs may be fused using a fiber sheet, and the fiber sheet may be applied to the subject to introduce the fused LNPs into the body of the subject.

For example, the first LNPs, derived from a subject, may be extracellular vesicles derived from a body fluid of the subject. The extracellular vesicles derived from a body fluid can be acquired using various methods. Examples of such methods may non-limitingly include methods such as ultracentrifugation, the use of a commercially available kit or equipment, the utilization of a microfluidic device, the utilization of nanowires, and the utilization of cellulose nanowires. The second LNPs may encapsulate an anticancer agent or an effective drug to be prescribed to the subject. The body fluid containing the extracellular vesicles or a solution derived from the body fluid may be absorbed or allowed to infiltrate into the fiber sheet or may be applied dropwise onto the fiber sheet.

In some embodiments, a solution containing the first LNPs and a solution containing the second LNPs may be absorbed into a fiber sheet. In some embodiments, second LNPs encapsulating an effective drug to be prescribed to a subject may be supported on a fiber sheet in advance. A solution derived from a body fluid of the subject containing extracellular vesicles may be absorbed into the fiber sheet supporting the second LNPs. For example, the fiber sheet supporting the second LNPs may be applied to the surface of the skin, a mucous membrane, or an organ (the skin, etc.) of the subject to allow the body fluid to be absorbed into the fiber sheet. For example, the body fluid, a liquid derived from the body fluid, or a culture medium may be applied dropwise onto the fiber sheet supporting the second LNPs. The acquisition of first LNPs derived from a subject and the fusion between them and second LNPs containing a drug may be performed using the same fiber sheet. The acquisition of the first LNPs and fusion may be performed efficiently within a short time or substantially simultaneously.

The fused LNPs may be dried and/or stored on the same fiber sheet or after being transferred to another fiber sheet. The fiber sheet retaining the fused LNPs may be applied to the surface of the skin, etc., of the subject. Through this, the fused LNPs are introduced or absorbed into the body of the subject at the site of application. The fiber sheet retaining the fused LNPs may be applied to the skin, etc., of the subject during the subject's surgery to allow the sheet to absorb a body fluid of the subject, thereby causing the LNPs to fuse and/or introducing the fused LNPs into the subject.

The fiber sheet retaining the first LNPs, the second LNPs, and/or the fused LNPs may be applied to the skin, etc., of the subject during surgery or after surgery. For example, a fiber sheet retaining second LNPs or fused LNPs containing an anticancer agent may be applied to the skin, etc., of a body area with suspected tumor metastasis or incomplete resection during surgery or after surgery. Through this, an effective reduction of the recurrence, metastasis, etc., of cancer, for example, can be achieved.

### <Embodiment 2>

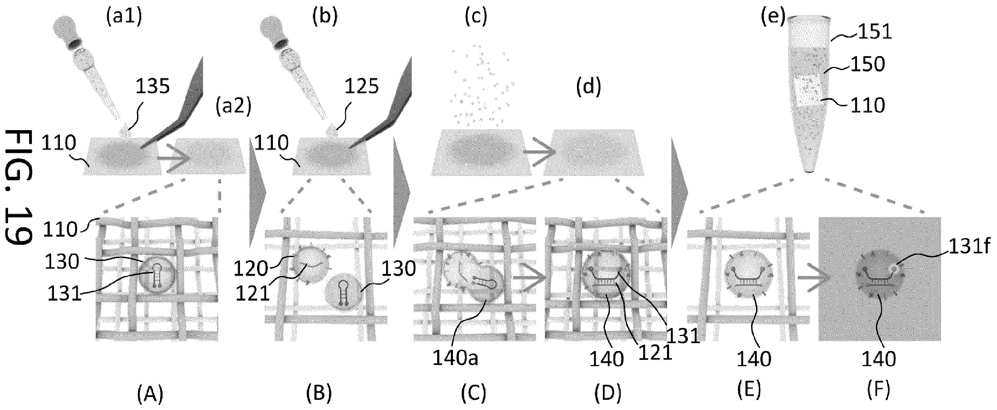

With reference to Fig. 19, a process according to an embodiment of the fusion of lipid nanoparticles through the contraction of a fibrous structure and the mixing and reaction between substances therein is schematically described. The upper diagrams, (a1) to (e), in Fig. 19 illustrate schematic views of the structure and other components in macroscopic view. The lower diagrams, (A) to (F), in Fig. 19 illustrate schematic views of the structure and other components in microscopic view, corresponding to diagrams (a1) to (e).

First, an aqueous solution 135 containing labeled LNPs 130, which encapsulate a molecular beacon 131 (Fig. 19A), is applied to the fibrous structure 110 (Fig. 19a1). Then the fibrous structure 110 is dried (Fig. 19a2). The labeled LNPs 130 are captured in the pores in the fibrous structure 110 (Fig. 19A). The molecular beacon 131 has been designed to hybridize with a target nucleic acid 121 encapsulated in target LNPs 120, which will be illustrated in the next drawing.

Then, to the fibrous structure 110, an aqueous solution 125 containing target LNPs 120, which encapsulate a target nucleic acid 121, is applied to the fibrous structure 110 (Fig. 19b). As a result, the fibrous structure 110 holds both the labeled LNPs 130 and the target LNPs 120 captured in it (Fig. 19B).

Drying this structure (Fig. 19c) causes the fibrous structure 110 to shrink and the pores to become smaller. The labeled LNPs 130 and the target LNPs 120 captured in them are compressed from the outside by the fibers in the fibrous structure 110. The LNPs come into contact with each other, initiating fusion and starting to form fused lipid nanoparticles 140a (Fig. 19C).

Allowing the structure to dry further (Fig. 19d) causes the pores to become even smaller. As a result of this, the labeled LNPs 130 and the target LNPs 120 completely fuse, and fused LNPs 140 are formed (Fig. 19D).

Inside the fused lipid nanoparticles 140, the molecular beacon 131 originally encapsulated in the target LNPs 130 binds to the target nucleic acid 121 originally encapsulated in the target LNPs 120 (Fig. 19D).

Placing this fibrous structure 110 into a container (tube) 151 containing a washing solution 150 and allowing the solution to stir (Fig. 19e) causes the pores in the fibrous structure 110 to open, allowing the fused LNPs 140 to be collected (Fig. 19E).

When the fused LNPs 140 obtained in this manner are subjected to fluorescence observation, fluorescence from the fluorophore 131f in the molecular beacon 131, which has hybridized with the target nucleic acid 121 within the fused LNPs 140, can be detected (Fig. 19F).

It should be noted that in this embodiment and the next embodiment, the fibrous structure is dried after the introduction of the labeled LNPs. The present disclosure, however, is not limited to this. After the introduction of the labeled LNPs, the target LNPs may be introduced without drying the fibrous structure or in a state in which the fibrous structure is wet. The fibrous structure may be dried for pore contraction after the introduction of both the labeled LNPs and the target LNPs. The order of introduction of the labeled LNPs and the target LNPs should not be construed with limitation to these embodiments either. The labeled LNPs may be introduced after the introduction of the target LNPs. The labeled LNPs and the target LNPs may be introduced simultaneously. LNPs may be introduced multiple times or introduced repeatedly.

### <EXAMPLE 5>

In this example, target LNPs encapsulating a target miRNA and labeled LNPs encapsulating a molecular beacon designed to hybridize with the target miRNA were separately produced by the same methods as described above. The target miRNA was nucleic acid miR1246. The molecular beacon in the labeled LNPs was designed to hybridize with this miR1246.

A PBS dispersion of the target LNPs, which encapsulated the target nucleic acid miR1246 (Hereinafter also referred to as "LNP(miR)."), and a PBS dispersion of the labeled LNPs, which encapsulated a molecular beacon having Cy5 as a fluorophore and intended to hybridize with miR1246 (Hereinafter also referred to as "LNP(MB)."), were each prepared. The concentrations of the LNPs were adjusted as appropriate. Ten microliters each of the dispersions were mixed, and the resulting mixture was applied to a 1 cm × 1 cm carbon nanofiber (CNF) sheet. Then the CNF sheet was dried for 2 days or longer within a desiccator. Through this, fused LNPs (Hereinafter also referred to as "(miR)+(MB).") were formed.

The dried CNF sheet was placed into a 1.5-mL tube and washed with 1 mL of PBS. The PBS washings were discarded, and 50 µL of PBS was added to immerse the CNF sheet. Stirring was performed using a vortex mixer to extract fused LNPs and residual LNPs.

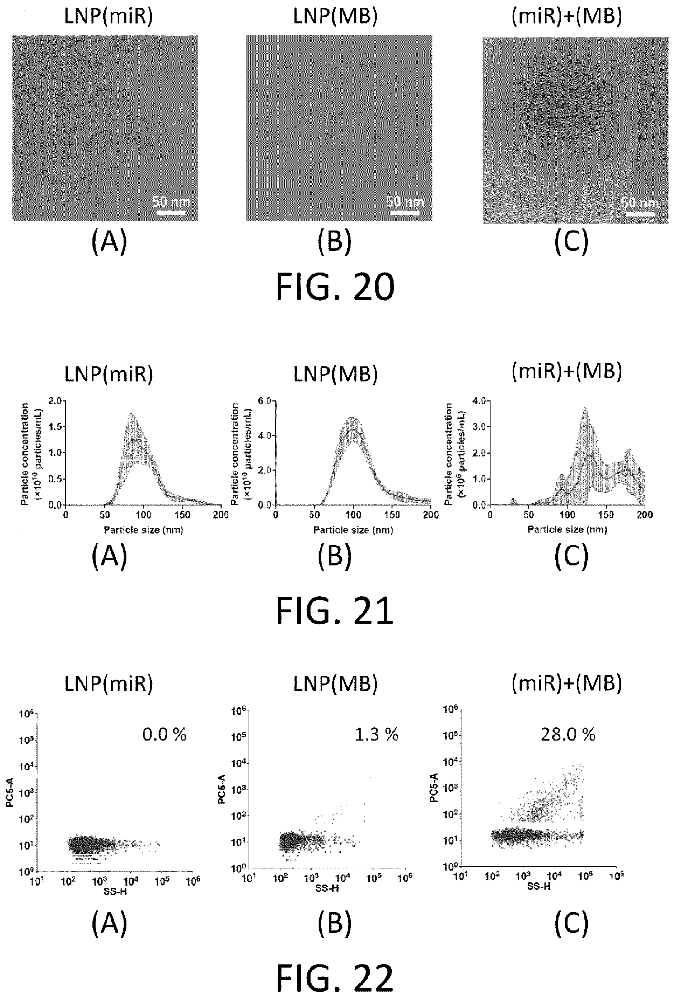

In Fig. 20, electron micrographs of LNP(miR) (Fig. 20A), LNP(MB) (Fig. 20B), and (miR)+(MB) (Fig. 20C) are presented. For (miR)+(MB) (Fig. 20C), LNPs larger than LNP(miR) (Fig. 20A) and LNP(MB) (Fig. 20B) were observed.

In Fig. 21, the distributions of particle sizes for LNP(miR) (Fig. 21A), LNP(MB) (Fig. 21B), and (miR)+(MB) (Fig. 21C) as measured by nano-tracking analysis are presented. LNP(miR) had a peak around about 90 nm (Fig. 21A), and LNP(MB) had a peak around about 100 nm (Fig. 21B). (miR)+(MB) (Fig. 21C), by contrast, was found to have several peaks, having the highest peak around 130 nm, and have a larger size than LNP(miR) (Fig. 21A) and LNP(MB) (Fig. 21B). That is, this indicates that the size increased as a result of fusion.

In Fig. 22, the results of flow cytometry for LNP(miR) (Fig. 22A), LNP(MB) (Fig. 22B), and (miR)+(MB) (Fig. 22C) are presented. For LNP(miR) (Fig. 22A), almost all measurement points were below at a fluorescence intensity of about 2 × 10¹. These points represent data that were counted as particles but not counted for the optical label. For LNP(MB) (Fig. 22B), only 1.3% were counted as fluorescent labels, with almost all measurement points being below at a fluorescence intensity of about 2 × 10¹. As for (miR)+(MB) (Fig. 22C), 28% were not noise; that is, they were detected as signals corresponding to the molecular beacon PC5, which was the target for measurement. Based on these, it was confirmed that as a result of fusion, the molecular beacon hybridized with miR1246 as its target within the fused LNPs.

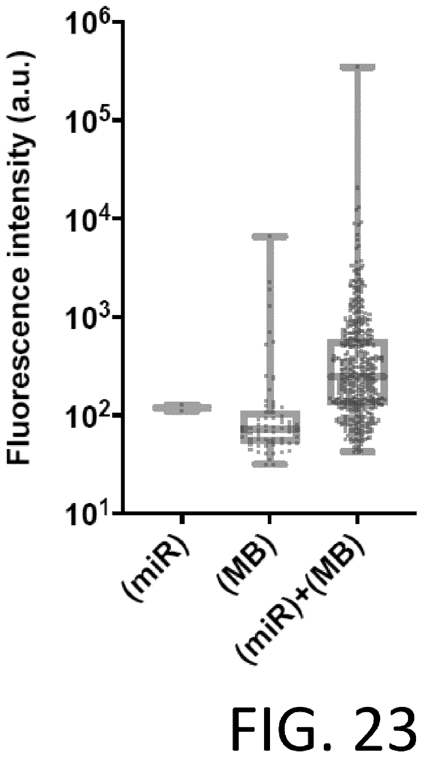

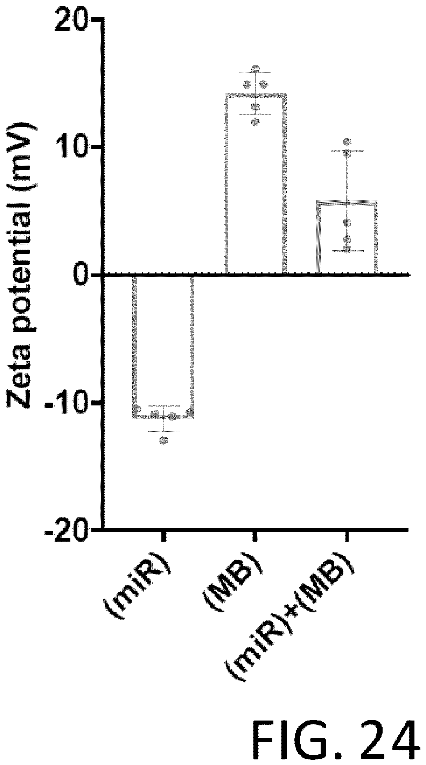

In Fig. 23, the fluorescence intensity of LNP(miR) (Fig. 23A), LNP(MB) (Fig. 23B), and (miR)+(MB) (Fig. 24C) as measured using a plate reader is presented. Based on these results, too, Based on this, it was confirmed that as a result of fusion, the molecular beacon hybridized with miR1246 as its target within the fused LNPs.

In Fig. 24, the results of surface potential (Zeta Potential (mV)) for LNP(miR), LNP(MB), and (miR)+(MB) are presented. The surface potentials of LNP(miR) and LNP(MB) were approximately -10 mV and approximately +15 mV, respectively. The surface potential of (miR)+(MB) after fusion, on the other hand, was approximately +5 mV. These results indicate that LNP(miR) and LNP(MB), both having negative charge, underwent membrane fusion through the fusion process.

### <EXAMPLE 6: Fusion between RPTEC-Derived EVs and LNP(MB)>

Fusion between EVs derived from human renal proximal tubule epithelial cells (RPTECs) and LNPs encapsulating a molecular beacon was performed. The molecular beacon was designed to hybridize with miR1246.

Cell cultivation and inflammation activation were performed as follows. EVs derived from activated RPTECs and EVs derived from non-activated RPTECs were prepared. First, RPTECs immortalized with TERT1 were cultured in Dulbecco's Modified Eagle Medium (DMEM) (High Glucose, 10% FBS, 1% penicillin/streptomycin). After 48 hours passed following seeding, the medium was washed with PBS. The resulting cells were cultured in Advanced DMEM with 1% penicillin, and the culture supernatant, which contained EVs, was collected 24 hours later. Through this, EVs derived from non-activated RPTECs were prepared. For inflammation deactivation, interleukins were added when switching to Advanced DMEM as described above. Specifically, 100 ng/mL of recombinant human IL-6, 100 ng/mL of recombinant human IL-6R, and 50 ng/mL of recombinant human IL-17 were added. Through this, EVs derived from activated RPTECs were prepared. For "miR1246" below, miR1246 was loaded into Lipofectamine, and this formulation was added when switching to Advanced DMEM as described above. For "miR1246+ILmix," miR1246 was loaded into Lipofectamine, and this formulation and IL-6, IL-6R, and IL-17 were added when switching to Advanced DMEM as described above.

Then purification of the EVs was performed. First, an RPTEC supernatant (approximately 50 mL) collected from three 15 cm × 15 cm dishes was centrifuged at 300×g and 4°C for 10 minutes to remove debris, such as cell debris. Then the supernatant was centrifuged at 2000×g and 4°C for 10 minutes and passed through a 0.22-µm filter (Millipore^{®}) to remove coarse particles. The supernatant, furthermore, was ultracentrifuged at 110000×g and 4°C for 80 minutes. Finally, it was concentrated to approximately 100 µL.

### <Role of miR1246 in IL6 Amplification>

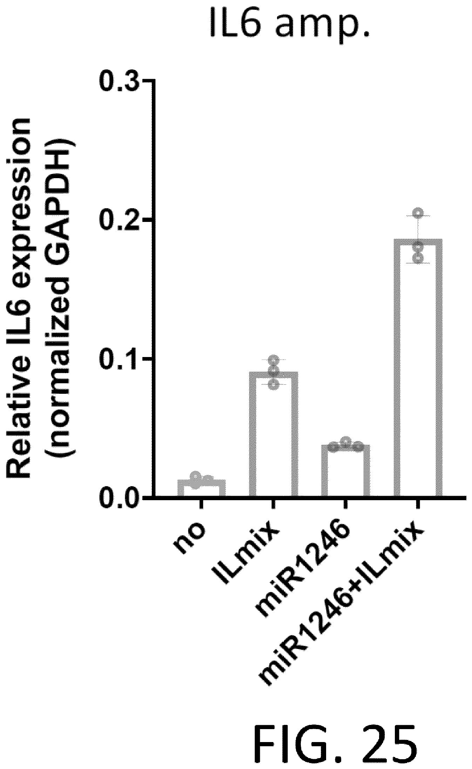

In Fig. 25, the expression of IL6 when no activation was performed ("no"), when IL-6, IL-6R, and IL-17 were added ("ILmix"), when miR1246 was added ("miR1246"), and when miR1246 was added with the addition of IL-6, IL-6R, and IL-17 ("miR1246+ILmix") is presented.

In the case of "ILmix," it was found that even with EVs derived from non-immune cells, RPTECs, IL6 is excessively expressed; IL6 amplification occurred. Even with the addition of miR1246 alone ("miR1246"), excessive expression was still observed. That is, it was found that miR1246 acts as a trigger for IL6 expression. For "miR1246+ILmix," it was found that IL6 amplification was further boosted.

As seen from these, it was found that miR1246 induces the expression of IL6. In some embodiments, miR1246 can be used as a marker for IL6 amplification.

### <Fusion Process>

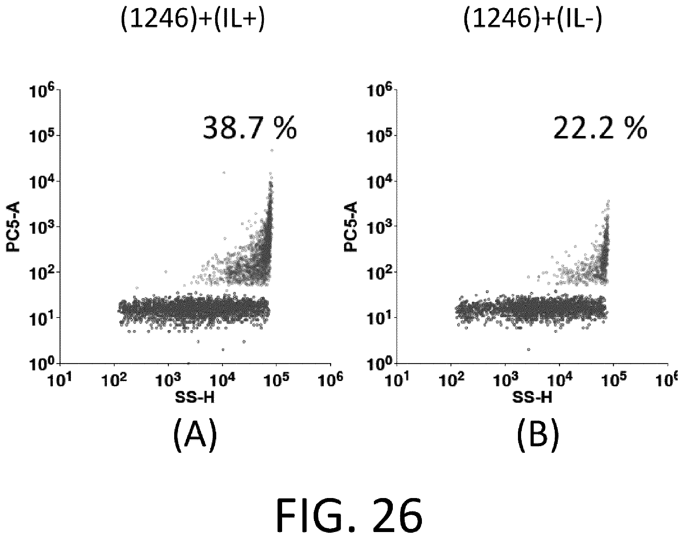

The fusion process was the same as in Example 5. In Fig. 26, the results of flow cytometry for LNPs fused from EVs derived from RPTECs activated with IL-6, IL-6R, and IL-17 (EV(IL+)) and LNP(1246MB) ((1246)+(IL+)) (Fig. 26A) and LNPs fused from EVs derived from non-activated RPTECs (IL-)) and LNP(1246MB) ((1246)+(IL-)) (Fig. 26B).

In the case without activation, presented in Fig. 26B ((1246)+(IL-)), 22.2% were detected as signals. miR1246 appears to have functions beyond IL6 amplification and is present even in the case without activation. In the case with activation, presented in Fig. 26A ((1246)+(IL+)), by contrast, 38.7% were detected as signals. This indicates that miR1246 expression increased as a result of activation, and the molecular beacon in the labeled LNPs recognized it through hybridization as a result of LNP fusion.

### <Embodiment 3>

In some embodiments, the target LNPs may be stained with a labeled antibody (e.g., a labeled antibody for immunofluorescence staining). As a result, the fused LNPs can have a stained portion in their protein membrane and a fluorophore in a molecular beacon inside. Accordingly, it is possible to detect and observe the fused LNPs by two types of optical measurement.

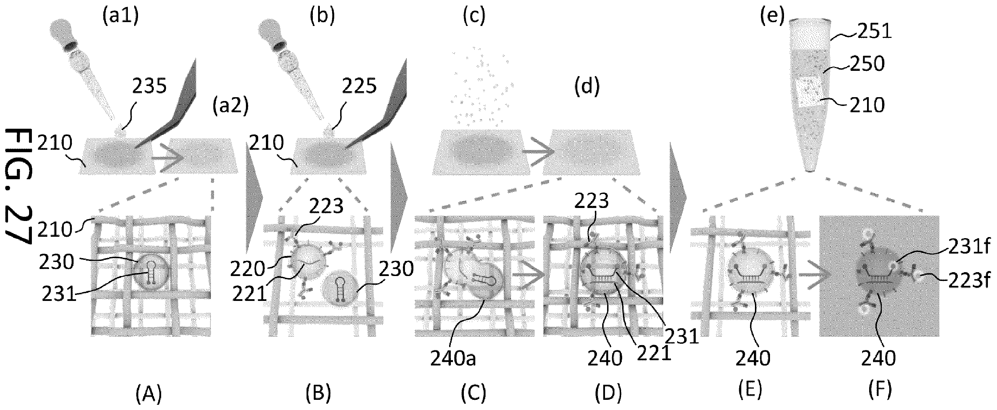

With reference to Fig. 27, a process according to an embodiment of the fusion of lipid nanoparticles through the contraction of a fibrous structure and the mixing and reaction between substances therein is schematically described. The upper diagrams, (a1) to (e), in Fig. 27 illustrate schematic views of the structure and other components in macroscopic view. The lower diagrams, (A) to (F), in Fig. 27 illustrate schematic views of the structure and other components in microscopic view, corresponding to diagrams (a1) to (e).

First, an aqueous solution 235 containing labeled LNPs 230, which encapsulate a molecular beacon 231 (Fig. 27A), is applied to the fibrous structure 210 (Fig. 27a1). Then the fibrous structure 210 is dried (Fig. 27a2). The labeled LNPs 230 are captured in the pores in the fibrous structure 210 (Fig. 27A). The molecular beacon 231 has been designed to hybridize with a target nucleic acid 221 encapsulated in target LNPs 220, which will be illustrated in the next drawing.

First, target LNPs 220, which encapsulate a target nucleic acid 221, are prepared, and a labeled antibody 223 is allowed to recognize the surface of their membrane protein (not illustrated). Then, to the fibrous structure 210, an aqueous solution 225 containing these target LNPs 220 is applied to the fibrous structure 210 (Fig. 27b). As a result, the fibrous structure 210 holds both the labeled LNPs 230 and the target LNPs 220 (Fig. 27B) captured in it.

Drying this structure (Fig. 27c) causes the fibrous structure 210 to shrink and the pores to become smaller. The labeled LNPs 230 and the target LNPs 220 captured in them are compressed from the outside by the fibers in the fibrous structure 210. The LNPs come into contact with each other, initiating fusion and starting to form fused lipid nanoparticles 240a (Fig. 27C).

Allowing the structure to dry further (Fig. 27d) causes the pores to become even smaller. As a result of this, the labeled LNPs 230 and the target LNPs 220 completely fuse, and fused LNPs 240 are formed (Fig. 27D).

Inside the fused lipid nanoparticles 240, the molecular beacon 231 originally encapsulated in the target LNPs 230 binds to the target nucleic acid 221 originally encapsulated in the target LNPs 220 (Fig. 27D). The membrane protein on the fused lipid nanoparticles 240 has the labeled antibody 223 derived from the target LNPs 220.

Placing this fibrous structure 210 into a container (tube) 251 containing a washing solution 250 and allowing the solution to stir (Fig. 27e) causes the pores in the fibrous structure 210 to open, allowing the fused LNPs 240 to be collected (Fig. 27E).

When the fused LNPs 240 obtained in this manner are subjected to fluorescence observation, fluorescence from the fluorophore 231f in the molecular beacon 231, which has hybridized with the target nucleic acid 221 within the fused LNPs 240, and fluorescence from the fluorescent label 223f, which has bound to the membrane protein, can be detected (Fig. 27F).

### <EXAMPLE 7 and EXAMPLE 8>

In Example 7, RPTEC-derived EVs were prepared in the same manner as in Example 5, and their membrane protein CD6 or LDHB (lactate dehydrogenase B) was stained with FITC (fluorescein isothiocyanate) in Example 8). Specifically, a labeled antibody for each was added to the prepared EVs to 100 nM, and the resulting mixture was incubated at 4°C overnight. Then the mixture was washed with PBS and ultracentrifuged. The fusion process is the same as in Example 5.

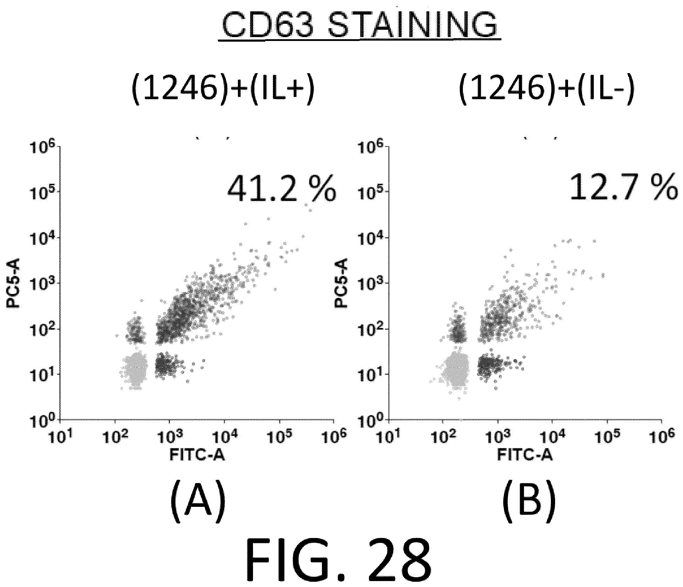

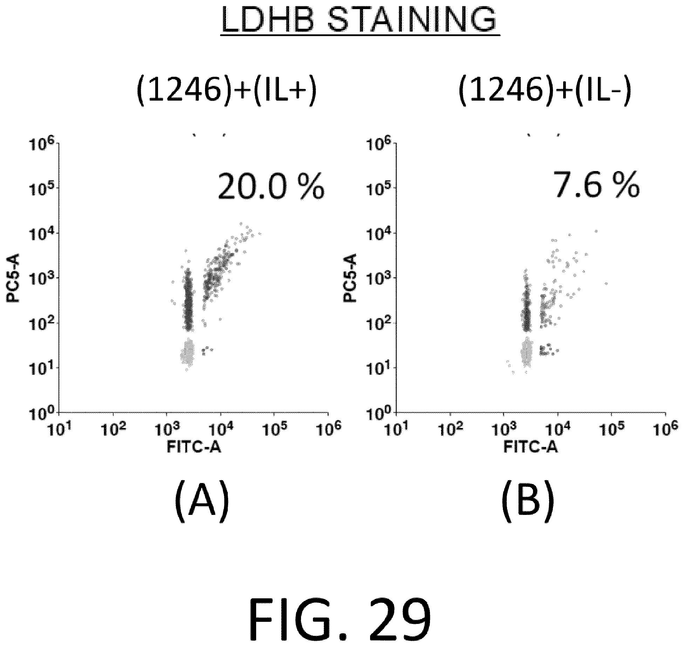

In Fig. 28, the results of flow cytometry in Example 7 are presented, and in Fig. 29, the results of flow cytometry in Example 8 are presented. The horizontal axis (FITC-A) indicates the fluorescence intensity of FITC modifying the membrane protein. The vertical axis (PC5-A) indicates the fluorescence intensity of the fluorophore PC5 in the molecular beacon. The % in the figures is the percentage of the portion detected as signals for both FITC as the label for the membrane protein and the fluorophore PC5 in the molecular beacon.

In both cases, the signal level increased through activation (41.2% in Fig. 28A and 20.0% in Fig. 29A) compared with that without activation (12.7% in Fig. 28B and 7.6% in Fig. 29B). This suggests both of the following: activation increased miR1246 expression, and also increased the expression of the membrane protein. As the expression of the membrane protein increases, miR expression may also possibly increase.

In some embodiments, the antibody label may be bound to multiple membrane proteins. This enables efficient detection of multiple membrane proteins. In some embodiments, the labeled LNPs may contain multiple molecular beacons that hybridize with different nucleic acids. In some embodiments, multiple types of labeled LNPs may be provided, and different molecular beacons may be encapsulated in each type of labeled LNP. For example, first labeled LNPs may encapsulate a first molecular beacon that hybridizes with a first nucleic acid, second labeled LNPs may encapsulate a second molecular beacon that hybridizes with a second nucleic acid, and they may be introduced into the target LNPs simultaneously. These embodiments enable efficient detection of multiple nucleic acids in the target LNPs. In some embodiments, these may be combined for simultaneous detection of multiple membrane proteins and multiple target nucleic acids.

By using such methods, a subject's disease and the site of the disease can be estimated or determined. From the characteristics of membrane proteins on the target LNPs, their site of origin can be estimated. From the types of nucleic acids encapsulated in the target LNPs, diseases can be estimated. For example, by detecting miR1246 present inside EVs acquired from a body fluid, such as blood, the types or the presence or absence of autoimmune diseases can be estimated from the presence or absence and/or degree of IL6 amplification. At the same time, from the types of membrane proteins on the EVs, the site of origin of the cells that secreted the EVs can be estimated. For example, by detecting CD81 as a membrane protein, it can be estimated whether the subject has rheumatoid arthritis, i.e., that the site is a joint.

The present disclosure also provides the following embodiments:
A001 A method for fusing lipid nanoparticles, the method including:
   providing a fibrous structure;
   providing a first solution containing a first lipid nanoparticle;
   providing a second solution containing a second lipid nanoparticle;
   causing the fibrous structure to absorb the first solution and capture the first lipid nanoparticle;
   causing the fibrous structure to absorb the second solution and capture the second lipid nanoparticle; and
   fusing the first lipid nanoparticle and the second lipid nanoparticle (and thereby forming a fused lipid nanoparticle) by causing the fibrous structure to contract.
A001b A method for fusing lipid nanoparticles, the method including:
   causing a fibrous structure to absorb a first solution containing a first lipid nanoparticle and a second solution containing a second lipid nanoparticle and capture the first lipid nanoparticle and the second lipid nanoparticle; and
   fusing the first lipid nanoparticle and the second lipid nanoparticle (and thereby forming a fused lipid nanoparticle) by causing the fibrous structure to contract (by removing at least part of water from the fibrous structure).
A001c A method for fusing lipid nanoparticles, the method including:
   causing a fibrous structure to capture a first lipid nanoparticle;
   causing the fibrous structure to capture a second lipid nanoparticle; and
   fusing the first lipid nanoparticle and the second lipid nanoparticle (and thereby forming a fused lipid nanoparticle) by causing the fibrous structure to contract.
A011 The method according to any of A001 to A001c or any embodiment, wherein
   the first lipid nanoparticle encapsulates a first substance,
      the second lipid nanoparticle encapsulates a second substance, and
   the fusing the first lipid nanoparticle and the second lipid nanoparticle (and thereby forming a fused lipid nanoparticle) includes mixing the first substance and the second substance and/or allowing the first substance and the second substance to react.
A012 The method according to any of A001 to A011 or any embodiment, wherein
   the first lipid nanoparticle and the second lipid nanoparticle have the same sign of charge.
A021 The method according to any of A001 to A012 or any embodiment, wherein
   the first lipid nanoparticle is an extracellular vesicle (EV) encapsulating a nucleic acid,
   the second lipid nanoparticle encapsulates a lipid nanoparticle encapsulating a molecular beacon that binds to (hybridizes with) a target sequence in the nucleic acid,
   the fusing the first lipid nanoparticle and the second lipid nanoparticle includes causing the molecular beacon to recognize the nucleic acid, and
   the method further includes
   measuring light emitted from the molecular beacon (or by a fluorophore in the molecular beacon).
A021b A method for estimating biological information of a subject, the method including:
   providing a first solution containing a first lipid nanoparticle derived from the subject;
   providing a second solution containing a second lipid nanoparticle encapsulating a molecular beacon that hybridizes with a nucleic acid potentially encapsulated in the first lipid nanoparticle;
   causing the fibrous structure to absorb the first solution and capture the first lipid nanoparticle;
   causing the fibrous structure to absorb the second solution and capture the second lipid nanoparticle;
   fusing the first lipid nanoparticle and the second lipid nanoparticle (and thereby forming a fused lipid nanoparticle) by causing the fibrous structure to contract, thereby allowing the molecular beacon to recognize the nucleic acid; and
   measuring light emitted by the molecular beacon present in the fused lipid nanoparticle.
A025 The method according to any of A001 to A021 or any embodiment, further including:
   labeling a membrane protein on the first lipid nanoparticle with a (e.g., optically) labeled antibody; and
   measuring the labeled antibody that the fused nanoparticle has.
A026 A method for estimating biological information of a subject, the method including:
   providing a first lipid nanoparticle derived from the subject;
   labeling a membrane protein on the first lipid nanoparticle with a (e.g., optically) labeled antibody (e.g., an antibody having an optical label);
   providing a first solution containing the labeled first lipid nanoparticle;
   providing a second solution containing a second lipid nanoparticle encapsulating a molecular beacon that hybridizes with a nucleic acid potentially encapsulated in the first lipid nanoparticle;
   causing the fibrous structure to absorb the first solution and capture the first lipid nanoparticle;
   causing the fibrous structure to absorb the second solution and capture the second lipid nanoparticle;
   fusing the first lipid nanoparticle and the second lipid nanoparticle (and thereby forming a fused lipid nanoparticle) by causing the fibrous structure to contract, thereby allowing the molecular beacon to recognize the nucleic acid; and
   measuring light emitted by the molecular beacon present in the fused lipid nanoparticle; and
   measuring the (optical) label on the membrane protein on the fused lipid nanoparticle.
A027 The method according to A026 or any embodiment, wherein
   the first lipid nanoparticle is an extracellular vesicle (EV) derived from the subject.
A028 The method according to A027 or any embodiment, wherein
   the labeled antibody is a fluorescently labeled antibody, and
   the measuring the labeled antibody includes measuring light emitted from the fluorescently labeled antibody.
A029 The method according to any of A027 or 028 or any embodiment, further including:
   estimating a disease of the subject from the measurement of light emitted from the molecular beacon; and
   estimating a site of the disease of the subject from the measurement of the labeled antibody.
A029b The method according to any of A027 or 028 or any embodiment, further including
   estimating one or both of a type and a site of a disease of the subject from any one, at least one, or both of the measurement of light emitted from the molecular beacon and the measurement of the labeled antibody.
A031 The method according to any of A001 to A012 or any embodiment, wherein
   the first lipid nanoparticle is an extracellular vesicle (EV) derived from a subject, and
   the second lipid nanoparticle is a drug.
A032 The method according to A31 or any embodiment, wherein
   the first lipid nanoparticle is an extracellular vesicle (EV) derived from a patient with cancer (subject), and
   the second lipid nanoparticle is an anticancer agent (drug) for the cancer.
A033 The method according to A032 or any embodiment, further including
   introducing the fused nanoparticle into the patient with cancer.
A041 The method according to any of A001 to A033 or any embodiment, wherein
   the first lipid nanoparticle and the second lipid nanoparticle have the same sign of charge.
B001 An artificial lipid nanoparticle including a molecular beacon that has a sequence complementary to a target nucleic acid encapsulated in a target extracellular vesicle (that hybridizes with the target nucleic acid).
B002 The artificial lipid nanoparticle according to B001 or any embodiment, wherein
   the target nucleic acid is a microRNA.
B011 An artificial lipid nanoparticle including a drug.
B012 The artificial lipid nanoparticle according to B011 or any embodiment, wherein
   the drug is an anticancer agent or includes an anticancer agent.
B013 The artificial lipid nanoparticle according to B001 or B002 or any embodiment, wherein
   the artificial lipid nanoparticle is configured to be fused with a target extracellular vesicle derived from a subject using a fibrous structure.
B101 The artificial lipid nanoparticle according to any of B001 to B013 or any embodiment, wherein the artificial lipid nanoparticle is a drug or DDS drug.
B102 Use of the artificial lipid nanoparticle according to any of B001 to B013 or any embodiment as a drug or DDS drug.

While preferred embodiments of the present invention have been presented and described herein, it will be obvious to those skilled in the art that such embodiments are provided solely by way of example. It is not intended that the present invention be limited by the specific examples provided herein. While the present invention has been described with reference to the above description, the descriptions and illustrations of the embodiments herein are not meant to be construed in a limiting sense. Numerous variations, changes, and substitutions will now occur to those skilled in the art without departing from the present invention. It shall be, furthermore, understood that all aspects of the present invention are not limited to the specific depictions, configurations, or relative proportions set forth herein, which depend upon a variety of conditions and variables. It should be understood that various alternatives to the embodiments of the present invention described herein may be used in implementing the present invention. It is, therefore, contemplated that the present invention shall also cover any such alternatives, modifications, variations, or equivalents. It is intended that the claims of the present application define the scope of the present invention and that methods and structures within the scope of these claims and their equivalents be covered thereby.

## Claims

1. A method for fusing lipid nanoparticles, the method comprising:
causing a fibrous structure to capture a first lipid nanoparticle;
causing the fibrous structure to capture a second lipid nanoparticle; and
fusing the first lipid nanoparticle and the second lipid nanoparticle by causing the fibrous structure to contract.

2. The method according to Claim 1, wherein
the first lipid nanoparticle encapsulates a first substance,
the second lipid nanoparticle encapsulates a second substance, and
the fusing the first lipid nanoparticle and the second lipid nanoparticle includes mixing the first substance and the second substance and/or allowing the first substance and the second substance to react.

3. The method according to Claim 1, wherein
the first lipid nanoparticle and the second lipid nanoparticle have the same sign of charge.

4. The method according to Claim 1, wherein
the first lipid nanoparticle is an extracellular vesicle (EV) encapsulating a nucleic acid,
the second lipid nanoparticle encapsulates a lipid nanoparticle encapsulating a molecular beacon that binds to (hybridizes with) a target sequence in the nucleic acid,
the fusing the first lipid nanoparticle and the second lipid nanoparticle includes causing the molecular beacon to recognize the nucleic acid, and
the method further includes
measuring light emitted from the molecular beacon.

5. The method according to Claim 1, wherein
the first lipid nanoparticle is an extracellular vesicle (EV) derived from a subject, and
the second lipid nanoparticle is a drug.

6. The method according to Claim 5, wherein
the first lipid nanoparticle is an extracellular vesicle (EV) derived from a patient with cancer (subject), and
the second lipid nanoparticle is an anticancer agent (drug) for the cancer.

7. An artificial lipid nanoparticle comprising a molecular beacon having a sequence complementary to a target nucleic acid encapsulated in a target extracellular vesicle.

8. The artificial lipid nanoparticle according to Claim 7, wherein
the target nucleic acid is a microRNA.

9. An artificial lipid nanoparticle comprising a drug.

10. The artificial lipid nanoparticle according to Claim 8, wherein
the drug is an anticancer agent or includes an anticancer agent.

11. The artificial lipid nanoparticle according to any one of Claims 7 to 10, wherein
the artificial lipid nanoparticle is configured to be fused with a target extracellular vesicle derived from a subject using a fibrous structure.

12. A method for estimating biological information of a subject, the method comprising:
providing a first solution containing a first lipid nanoparticle derived from the subject;
providing a second solution containing a second lipid nanoparticle encapsulating a molecular beacon that hybridizes with a nucleic acid potentially encapsulated in the first lipid nanoparticle;
causing the fibrous structure to absorb the first solution and capture the first lipid nanoparticle;
causing the fibrous structure to absorb the second solution and capture the second lipid nanoparticle;
fusing the first lipid nanoparticle and the second lipid nanoparticle, and thereby forming a fused lipid nanoparticle, by causing the fibrous structure to contract, thereby allowing the molecular beacon to recognize the nucleic acid; and
measuring light emitted by the molecular beacon present in the fused lipid nanoparticle.

13. A method for estimating biological information of a subject, the method comprising:
providing a first lipid nanoparticle derived from the subject;
labeling a membrane protein on the first lipid nanoparticle with an optically labeled antibody;
providing a first solution containing the labeled first lipid nanoparticle;
providing a second solution containing a second lipid nanoparticle encapsulating a molecular beacon that hybridizes with a nucleic acid potentially encapsulated in the first lipid nanoparticle;
causing the fibrous structure to absorb the first solution and capture the first lipid nanoparticle;
causing the fibrous structure to absorb the second solution and capture the second lipid nanoparticle;
fusing the first lipid nanoparticle and the second lipid nanoparticle, and thereby forming a fused lipid nanoparticle, by causing the fibrous structure to contract, thereby allowing the molecular beacon to recognize the nucleic acid; and
measuring light emitted by the molecular beacon present in the fused lipid nanoparticle; and
measuring the optical label on the membrane protein on the fused lipid nanoparticle.

14. The method according to Claim 12 or 13, wherein
the first lipid nanoparticle is an extracellular vesicle (EV) derived from the subject.

15. The method according to Claim 12, further comprising
estimating one or both of a type and a site of a disease of the subject from the measurement of light emitted from the molecular beacon.

16. The method according to Claim 13, further comprising
estimating one or both of a type and a site of a disease of the subject from one or both of the measurement of light emitted from the molecular beacon and the measurement of the labeled antibody.
